(19) Europäisches Patentamt
European Patent Office
Office européen des brevets

(11) EP 1 369 479 A1

(12) **EUROPEAN PATENT APPLICATION**
published in accordance with Art. 158(3) EPC

(43) Date of publication:
**10.12.2003 Bulletin 2003/50**

(21) Application number: **02712400.7**

(22) Date of filing: **15.02.2002**

(51) Int Cl.[7]: **C12N 15/09**, C12N 15/12, C07K 14/47, C07K 16/18, C12N 1/15, C12N 1/19, C12N 1/21, C12N 5/10, C12Q 1/02, A61K 48/00, G01N 33/50

(86) International application number:
**PCT/JP02/01321**

(87) International publication number:
**WO 02/064771 (22.08.2002 Gazette 2002/34)**

(84) Designated Contracting States:
**AT BE CH CY DE DK ES FI FR GB GR IE IT LI LU MC NL PT SE TR**
Designated Extension States:
**AL LT LV MK RO SI**

(30) Priority: **15.02.2001 JP 2001039196**

(71) Applicant: **MOCHIDA PHARMACEUTICAL CO., LTD.**
**Shinjuku-ku Tokyo 160-8515 (JP)**

(72) Inventors:
- **NAKAMURA, Yusuke**
  **Yokohama-shi, Kanagawa 225-0011 (JP)**
- **SUGANO, Sumio**
  **Suginami-ku, Tokyo 167-0052 (JP)**
- **KATO, Yutaka,**
  **c/o MOCHIDA PHARMACEUTICAL CO. LTD.**
  **Shinjuku-ku, Tokyo 160-8515 (JP)**
- **TAKAHASHI, Tomohiro,**
  **c/o MOCHIDA PHARM. CO. LTD.**
  **Shinjuku-ku, Tokyo 160-8 515 (JP)**
- **SHIRAKAWA, Kamon,**
  **c/o MOCHIDA PHARM. CO. LTD.**
  **Shinjuku-ku, Tokyo 160-8515 (JP)**

(74) Representative: **Wablat, Wolfgang, Dr.Dr.**
**Patentanwalt,**
**Potsdamer Chaussee 48**
**14129 Berlin (DE)**

(54) **NOVEL CELL ADHESION MOLECULE SPECIFIC TO ACTIVATED LEUKOCYTE**

(57) A gene of a novel cellular adhesion molecule participating in the regulation of immune functions and a protein, and a method of efficiently evaluating an activity regulator for this protein are provided.

A DNA comprising the nucleotide sequence represented by SEQ ID No:1; a cellular adhesion molecule encoded by this DNA; an antisense nucleic acid to this DNA sequence; and a method of evaluating an activity regulator for this protein.

EP 1 369 479 A1

SEQUENCE LISTING

<110> Mochida Pharmaceutical Co. Ltd.

<120> Novel cellular adhesion molecule of activated leukocyte

<130> SAP-685-PCT

<160> 5

<170> PatentIn Ver. 2.1

<210> 1

<211> 984

<212> DNA

<400> 1

```
atggaaaagt ccatctggct gctggcctgc ttggcgtggg ttctcccgac aggctcattt  60
gtgagaacta aaatagatac tacggagaac ttgctcaaca cagaggtgca cagctcgcca 120
gcgcagcgct ggtccatgca ggtgccaccc gaggtgagcg cggaggcagg cgacgcggca 180
gtgctgccct gcaccttcac gcacccgcac cgccactacg acgggccgct gacggccatc 240
tggcgcgcgg gcgagcccta tgcgggcccg caggtgttcc gctgcgctgc ggcgcggggc 300
agcgagctct gccagacggc gctgagcctg cacggccgct tccggctgct gggcaacccg 360
cgccgcaacg acctctcgct gcgcgtcgag cgcctcgccc tggctgacga ccgccgctac 420
ttctgccgcg tcgagttcgc cggcgacgtc catgaccgct acgagagccg ccacggcgtc 480
cggctgcacg tgacagccgc gccgcggatc gtcaacatct cggtgctgcc cagtccggct 540
cacgccttcc gcgcgctctg cactgccgaa ggggagccgc cgcccgccct cgcctggtcc 600
ggcccggccc tgggcaacag cttggcagcc gtgcggagcc cgcgtgaggg tcacggccac 660
ctagtgaccg ccgaactgcc cgcactgacc catgacggcc gctacacgtg tacggccgcc 720
aacagcctgg gccgctccga ggccagcgtc tacctgttcc gcttccatgg cgccagcggg 780
gcctcgacgg tcgccctcct gctcggcgct ctcggcttca aggcgctgct gctgctcggg 840
gtcctggccg cccgcgctgc ccgccgccgc ccagagcatc tggacacccc ggacacccca 900
ccacggtccc aggcccagga gtccaattat gaaaatttga gccagatgaa cccccggagc 960
ccaccagcca ccatgtgctc accg                                       984
```

<210> 2

<211> 328

<212> PRT

<400> 2

```
Met Glu Lys Ser Ile Trp Leu Leu Ala Cys Leu Ala Trp Val Leu Pro
  1               5                  10                  15
```

Thr Gly Ser Phe Val Arg Thr Lys Ile Asp Thr Thr Glu Asn Leu Leu
20 25 30
Asn Thr Glu Val His Ser Ser Pro Ala Gln Arg Trp Ser Met Gln Val
35 40 45
Pro Pro Glu Val Ser Ala Glu Ala Gly Asp Ala Ala Val Leu Pro Cys
50 55 60
Thr Phe Thr His Pro His Arg His Tyr Asp Gly Pro Leu Thr Ala Ile
65 70 75 80
Trp Arg Ala Gly Glu Pro Tyr Ala Gly Pro Gln Val Phe Arg Cys Ala
85 90 95
Ala Ala Arg Gly Ser Glu Leu Cys Gln Thr Ala Leu Ser Leu His Gly
100 105 110
Arg Phe Arg Leu Leu Gly Asn Pro Arg Arg Asn Asp Leu Ser Leu Arg
115 120 125
Val Glu Arg Leu Ala Leu Ala Asp Asp Arg Arg Tyr Phe Cys Arg Val
130 135 140
Glu Phe Ala Gly Asp Val His Asp Arg Tyr Glu Ser Arg His Gly Val
145 150 155 160
Arg Leu His Val Thr Ala Ala Pro Arg Ile Val Asn Ile Ser Val Leu
165 170 175
Pro Ser Pro Ala His Ala Phe Arg Ala Leu Cys Thr Ala Glu Gly Glu
180 185 190
Pro Pro Pro Ala Leu Ala Trp Ser Gly Pro Ala Leu Gly Asn Ser Leu
195 200 205
Ala Ala Val Arg Ser Pro Arg Glu Gly His Gly His Leu Val Thr Ala
210 215 220
Glu Leu Pro Ala Leu Thr His Asp Gly Arg Tyr Thr Cys Thr Ala Ala
225 230 235 240
Asn Ser Leu Gly Arg Ser Glu Ala Ser Val Tyr Leu Phe Arg Phe His
245 250 255
Gly Ala Ser Gly Ala Ser Thr Val Ala Leu Leu Leu Gly Ala Leu Gly
260 265 270
Phe Lys Ala Leu Leu Leu Leu Gly Val Leu Ala Ala Arg Ala Ala Arg
275 280 285
Arg Arg Pro Glu His Leu Asp Thr Pro Asp Thr Pro Pro Arg Ser Gln
290 295 300

Ala Gln Glu Ser Asn Tyr Glu Asn Leu Ser Gln Met Asn Pro Arg Ser
305 310 315 320
Pro Pro Ala Thr Met Cys Ser Pro
325

<210> 3
<211> 1523
<212> DNA
<400> 3

```
tccggctccc gcagagccca cagggacctg cagatctgag tgccctgccc acccccgccc   60
gccttccttc ccccaccacg cctgggaggg ccctcactgg ggaggtggcc gagaacgggt  120
ctggcctggg gtgttcagat gctcacagca tggaaaagtc catctggctg ctggcctgct  180
tggcgtgggt tctcccgaca ggctcatttg tgagaactaa aatagatact acggagaact  240
tgctcaacac agaggtgcac agctcgccag cgcagcgctg gtccatgcag gtgccacccg  300
aggtgagcgc ggaggcaggc gacgcggcag tgctgccctg caccttcacg cacccgcacc  360
gccactacga cgggccgctg acggccatct ggcgcgcggg cgagccctat gcgggcccgc  420
aggtgttccg ctgcgctgcg gcgcggggca gcgagctctg ccagacggcg ctgagcctgc  480
acggccgctt ccggctgctg ggcaacccgc gccgcaacga cctctcgctg cgcgtcgagc  540
gcctcgccct ggctgacgac cgccgctact tctgccgcgt cgagttcgcc ggcgacgtcc  600
atgaccgcta cgagagccgc cacggcgtcc ggctgcacgt gacagccgcg ccgcggatcg  660
tcaacatctc ggtgctgccc agtccggctc acgccttccg cgcgctctgc actgccgaag  720
gggagccgcc gcccgccctc gcctggtccg gcccggccct gggcaacagc ttggcagccg  780
tgcggagccc gcgtgagggt cacggccacc tagtgaccgc cgaactgccc gcactgaccc  840
atgacggccg ctacacgtgt acggccgcca acagcctggg ccgctccgag gccagcgtct  900
acctgttccg cttccatggc gccagcgggg cctcgacggt cgccctcctg ctcggcgctc  960
tcggcttcaa ggcgctgctg ctgctcgggg tcctggccgc ccgcgctgcc cgccgccgcc 1020
cagagcatct ggacaccccg gacaccccac cacggtccca ggcccaggag tccaattatg 1080
aaaatttgag ccagatgaac cccggagccc caccagccac catgtgctca ccgtgaggag 1140
tccctcagcc accaacatcc atttcagcac tgtaaagaac aaaggccagt gcgaggcttg 1200
gctggcacag ccagtcctgg ttctcgggca ccttggcagc ccccagctgg gtggctcctc 1260
ccctgctcaa ggtcaagacc ctgctcaagg aggctcatct ggcctcctat gtggacaacc 1320
atttcggagc tccctgatat ttttgccagc atttcgtaaa tgtgcatacg tctgtgtgtg 1380
tgtgtgtgtg tgagagagag agagagagag tacacgcatt agcttgagcg tgaaacttcc 1440
agaaatgttc ccttgccctt tcttacctag aacacctgct atagtaaagc agacaggaaa 1500
ctgttaaaaa aaaaaaaaaa aaa                                         1523
```

<210> 4
<211> 762
<212> DNA
<400> 4

```
atggaaaagt ccatctggct gctggcctgc ttggcgtggg ttctcccgac aggctcattt  60
gtgagaacta aaatagatac tacggagaac ttgctcaaca cagaggtgca cagctcgcca 120
gcgcagcgct ggtccatgca ggtgccaccc gaggtgagcg cggaggcagg cgacgcggca 180
gtgctgccct gcaccttcac gcacccgcac cgccactacg acgggccgct gacggccatc 240
tggcgcgcgg gcgagcccta tgcgggcccg caggtgttcc gctgcgctgc ggcgcggggc 300
agcgagctct gccagacggc gctgagcctg cacggccgct tccggctgct gggcaacccg 360
cgccgcaacg acctctcgct gcgcgtcgag cgcctcgccc tggctgacga ccgccgctac 420
ttctgccgcg tcgagttcgc cggcgacgtc catgaccgct acgagagccg ccacggcgtc 480
cggctgcacg tgacagccgc gccgcggatc gtcaacatct cggtgctgcc cagtccggct 540
cacgccttcc gcgcgctctg cactgccgaa ggggagccgc cgcccgccct cgcctggtcc 600
ggcccggccc tgggcaacag cttggcagcc gtgcggagcc cgcgtgaggg tcacggccac 660
ctagtgaccg ccgaactgcc cgcactgacc catgacggcc gctacacgtg tacggccgcc 720
aacagcctgg gccgctccga ggccagcgtc tacctgttcc gc                   762
```

<210> 5
<211> 254
<212> PRT
<400> 5

```
Met Glu Lys Ser Ile Trp Leu Leu Ala Cys Leu Ala Trp Val Leu Pro
 1               5                  10                  15
Thr Gly Ser Phe Val Arg Thr Lys Ile Asp Thr Thr Glu Asn Leu Leu
            20                  25                  30
Asn Thr Glu Val His Ser Ser Pro Ala Gln Arg Trp Ser Met Gln Val
        35                  40                  45
Pro Pro Glu Val Ser Ala Glu Ala Gly Asp Ala Ala Val Leu Pro Cys
    50                  55                  60
Thr Phe Thr His Pro His Arg His Tyr Asp Gly Pro Leu Thr Ala Ile
65                  70                  75                  80
Trp Arg Ala Gly Glu Pro Tyr Ala Gly Pro Gln Val Phe Arg Cys Ala
                85                  90                  95
Ala Ala Arg Gly Ser Glu Leu Cys Gln Thr Ala Leu Ser Leu His Gly
            100                 105                 110
```

Arg Phe Arg Leu Leu Gly Asn Pro Arg Arg Asn Asp Leu Ser Leu Arg
115 120 125
Val Glu Arg Leu Ala Leu Ala Asp Asp Arg Arg Tyr Phe Cys Arg Val
130 135 140
Glu Phe Ala Gly Asp Val His Asp Arg Tyr Glu Ser Arg His Gly Val
145 150 155 160
Arg Leu His Val Thr Ala Ala Pro Arg Ile Val Asn Ile Ser Val Leu
165 170 175
Pro Ser Pro Ala His Ala Phe Arg Ala Leu Cys Thr Ala Glu Gly Glu
180 185 190
Pro Pro Pro Ala Leu Ala Trp Ser Gly Pro Ala Leu Gly Asn Ser Leu
195 200 205
Ala Ala Val Arg Ser Pro Arg Glu Gly His Gly His Leu Val Thr Ala
210 215 220
Glu Leu Pro Ala Leu Thr His Asp Gly Arg Tyr Thr Cys Thr Ala Ala
225 230 235 240
Asn Ser Leu Gly Arg Ser Glu Ala Ser Val Tyr Leu Phe Arg
245 250

<210> 6
<211> 279
<212> PRT
<400> 6
Met Glu Lys Ser Ile Trp Leu Leu Ala Cys Leu Ala Trp Val Leu Pro
1 5 10 15
Thr Gly Ser Phe Val Arg Thr Lys Ile Asp Thr Thr Glu Asn Leu Leu
20 25 30
Asn Thr Glu Val His Ser Ser Pro Ala Gln Arg Trp Ser Met Gln Val
35 40 45
Pro Pro Glu Val Ser Ala Glu Ala Gly Asp Ala Ala Val Leu Pro Cys
50 55 60
Thr Phe Thr His Pro His Arg His Tyr Asp Gly Pro Leu Thr Ala Ile
65 70 75 80
Trp Arg Ala Gly Glu Pro Tyr Ala Gly Pro Gln Val Phe Arg Cys Ala
85 90 95
Ala Ala Arg Gly Ser Glu Leu Cys Gln Thr Ala Leu Ser Leu His Gly

100 105 110
Arg Phe Arg Leu Leu Gly Asn Pro Arg Arg Asn Asp Leu Ser Leu Arg
115 120 125
Val Glu Arg Leu Ala Leu Ala Asp Asp Arg Arg Tyr Phe Cys Arg Val
130 135 140
Glu Phe Ala Gly Asp Val His Asp Arg Tyr Glu Ser Arg His Gly Val
145 150 155 160
Arg Leu His Val Thr Ala Ala Pro Arg Ile Val Asn Ile Ser Val Leu
165 170 175
Pro Ser Pro Ala His Ala Phe Arg Ala Leu Cys Thr Ala Glu Gly Glu
180 185 190
Pro Pro Pro Ala Leu Ala Trp Ser Gly Pro Ala Leu Gly Asn Ser Leu
195 200 205
Ala Ala Val Arg Ser Pro Arg Glu Gly His Gly His Leu Val Thr Ala
210 215 220
Glu Leu Pro Ala Leu Thr His Asp Gly Arg Tyr Thr Cys Thr Ala Ala
225 230 235 240
Asn Ser Leu Gly Arg Ser Glu Ala Ser Val Tyr Leu Ala Arg Gly His
245 250 255
Pro Phe Glu Gln Lys Leu Ile Ser Glu Glu Asp Leu Asn Met His Thr
260 265 270
Gly His His His His His His
275

<210> 7
<211> 489
<212> PRT
<400> 7
Met Glu Lys Ser Ile Trp Leu Leu Ala Cys Leu Ala Trp Val Leu Pro
1 5 10 15
Thr Gly Ser Phe Val Arg Thr Lys Ile Asp Thr Thr Glu Asn Leu Leu
20 25 30
Asn Thr Glu Val His Ser Ser Pro Ala Gln Arg Trp Ser Met Gln Val
35 40 45
Pro Pro Glu Val Ser Ala Glu Ala Gly Asp Ala Ala Val Leu Pro Cys
50 55 60

Thr Phe Thr His Pro His Arg His Tyr Asp Gly Pro Leu Thr Ala Ile
65 70 75 80
Trp Arg Ala Gly Glu Pro Tyr Ala Gly Pro Gln Val Phe Arg Cys Ala
85 90 95
Ala Ala Arg Gly Ser Glu Leu Cys Gln Thr Ala Leu Ser Leu His Gly
100 105 110
Arg Phe Arg Leu Leu Gly Asn Pro Arg Arg Asn Asp Leu Ser Leu Arg
115 120 125
Val Glu Arg Leu Ala Leu Ala Asp Asp Arg Arg Tyr Phe Cys Arg Val
130 135 140
Glu Phe Ala Gly Asp Val His Asp Arg Tyr Glu Ser Arg His Gly Val
145 150 155 160
Arg Leu His Val Thr Ala Ala Pro Arg Ile Val Asn Ile Ser Val Leu
165 170 175
Pro Ser Pro Ala His Ala Phe Arg Ala Leu Cys Thr Ala Glu Gly Glu
180 185 190
Pro Pro Pro Ala Leu Ala Trp Ser Gly Pro Ala Leu Gly Asn Ser Leu
195 200 205
Ala Ala Val Arg Ser Pro Arg Glu Gly His Gly His Leu Val Thr Ala
210 215 220
Glu Leu Pro Ala Leu Thr His Asp Gly Arg Tyr Thr Cys Thr Ala Ala
225 230 235 240
Asn Ser Leu Gly Arg Ser Glu Ala Ser Val Tyr Leu Gly Ser Arg Ser
245 250 255
Asn Glu Pro Lys Ser Cys Asp Lys Thr His Thr Cys Pro Pro Cys Pro
260 265 270
Ala Pro Glu Leu Leu Gly Gly Pro Ser Val Phe Leu Phe Pro Pro Lys
275 280 285
Pro Lys Asp Thr Leu Met Ile Ser Arg Thr Pro Glu Val Thr Cys Val
290 295 300
Val Val Asp Val Ser His Glu Asp Pro Glu Val Lys Phe Asn Trp Tyr
305 310 315 320
Val Asp Gly Val Glu Val His Asn Ala Lys Thr Lys Pro Arg Glu Glu
325 330 335
Gln Tyr Asn Ser Thr Tyr Arg Val Val Ser Val Leu Thr Val Leu His
340 345 350

Gln Asp Trp Leu Asn Gly Lys Glu Tyr Lys Cys Lys Val Ser Asn Lys
355 360 365

Ala Leu Pro Ala Pro Ile Glu Lys Thr Ile Ser Lys Ala Lys Gly Gln
370 375 380

Pro Arg Glu Pro Gln Val Tyr Thr Leu Pro Pro Ser Arg Asp Glu Leu
385 390 395 400

Thr Lys Asn Gln Val Ser Leu Thr Cys Leu Val Lys Gly Phe Tyr Pro
405 410 415

Ser Asp Ile Ala Val Glu Trp Glu Ser Asn Gly Gln Pro Glu Asn Asn
420 425 430

Tyr Lys Thr Thr Pro Pro Val Leu Asp Ser Asp Gly Ser Phe Phe Leu
435 440 445

Tyr Ser Lys Leu Thr Val Asp Lys Ser Arg Trp Gln Gln Gly Asn Val
450 455 460

Phe Ser Cys Ser Val Met His Glu Ala Leu His Asn His Tyr Thr Gln
465 470 475 480

Lys Ser Leu Ser Leu Ser Pro Gly Lys
485

## Description

### FIELD OF THE INVENTION

The present invention relates to a novel cellular adhesion molecule specifically expressing on activated leukocytes and its partial peptide, a DNA encoding this protein and its fragment, a recombinant vector including this DNA, a transformant produced by transformation with this recombinant vector, a method of screening an agent capable of regulating an activity of this protein, an antibody having a reactivity with this protein or its partial peptide, a method of determining this protein using this antibody, and a recombinant non-human animal.

### BACKGROUND OF THE INVENTION

Cells control their functions, proliferation and differentiation by communicating with any other cells. Among cellular adhesion molecules, a cellular adhesion molecule of leukocytes significantly participating in all immune responses *in vivo* is important since it can regulate immune functions per se.

A sialoadhesin family is a cellular adhesion molecule family which recognizes a sequence of a sialic acid-containing sugar chain different from a selectin family and having an immunoglobulin-like domain structure. Until now, CD22 (Immunol. Today, Vol. 15, pp. 442-449 (1994)), sialoadhesin (Curr. Biol., Vol. 4, pp. 965-972 (1994)), MAG (myelin-associated glycoprotein) (Neuron, Vol. 13, pp. 229-246 (1994)), CD33 (Blood, Vol. 85, pp. 2005-2012 (1995)), AIRM1 (J. Exp. Med., Vol. 190, pp. 793-801 (1999)) and the like have been known. This sialoadhesin family is a transmembrane protein of type I whose N terminal directs extracellularly and whose extracellular region comprises one V-type immunoglobulin domain and plural C2-type immunoglobulin domains (see Fig. 1). The V domain comprises nine (A, B, C, C', C", D, E, F and G) β strands, two (GFCC'C" and ABED) β sheet structures forming a structure crosslinked with a disulfide bond between B and E strands . From the result of the experiment introducing CD22 and sialoadhesin in the V domain as point mutagens, it was shown that an arginine residue of the F strand conserved between sialoadhesin family molecules is essential for a sialic acid dependent bond. Each molecule expresses cell specifically and it participates in a cellular adhesion via a sialated sugar chain bond. For example, AIRM1 expresses on NK cells. CD33 expresses on precursor cells of bone marrow. They have receptor functions inhibiting the activation of NK cells and the differentiation into dendritic cells, respectively. It is known that CD22, sialoadhesin and MAG specifically express on mature B cells, macrophages and myelinating cells, respectively.

As mentioned above, recently many factors participating in the cellular adhesion of leukocytes have been known, but the isolation and identification of a novel cellular adhesion molecule participating in complicated immune response and inflammatory response *in vivo* are desired in order to elucidate the immune response and the inflammatory response.

An object of the present invention is to identify a novel adhesion molecule specifically expression on activated leukocytes and its gene and thereby to provide a medicine and a method useful in the prevention and the treatment of immune diseases.

### SUMMARY OF THE INVENTION

The present invention relates to a novel gene (hrc12337) and a novel cellular adhesion molecule (HRC12337) encoded by said gene. And, the present invention relates to a recombinant vector including said DNA, a transformant produced by transformation with said recombinant vector, a method of screening an activity regulator for said protein, an antibody having a reactivity with said protein or its partial peptide, a method of determining said protein using said antibody, and a gene transferred non-human animal.

<nucleic acid>

The present invention provides a gene hrc12337 encoding HRC12337 as described below in more detail. Specifically, the gene hrc12337 means a DNA encoding a cellular adhesion molecule which comprises the amino acid sequence represented by SEQ ID NO:2. It includes a cDNA shown in SEQ ID NO:1 and SEQ ID NO:3 and a genomic DNA that said cDNA comes from. Although this gene can be isolated and identified from a human renal cortical epithelial cell, the gene may be a DNA obtained by cloning using a genetic engineering technique such as a hybridization or a chemical synthetic technique such as a phosphoramidite method based on the sequence as disclosed herein. The form of the gene may be a cDNA, a genomic DNA and a chemically synthesized DNA, however not limited thereto.

The DNA of the present invention may be a single strand DNA. Alternatively, it may bind to a DNA or an RNA having the sequence complementary thereto to form a double-or triple-strand. The DNA may be labeled with an enzyme such as horseradish peroxidase (HRP); a radioactive isotope; a fluorescent substance; a chemiluminescent substance;

and the like.

If the nucleotide sequence of hrc12337 is provided, a sequence of an RNA and a sequence of a complementary DNA and RNA are univocally determined. Therefore, it should be understood that the present invention also provides an RNA corresponding to the DNA of the present invention as well as a DNA and an RNA having a sequence complementary to the DNA of the present invention. "DNA" and "polynucleotide" are interchangeably used herein.

The DNA of the present invention also includes a DNA hybridizable with the DNA comprising the nucleotide sequence represented by SEQ ID No. 1 under stringent conditions.

Variations of the nucleotide sequence represented by SEQ ID NO:1 are acceptable as long as they are hybridizable with the DNA comprising the nucleotide sequence represented by SEQ ID NO:1 under stringent conditions and a protein encoded by said DNA is a cellular adhesion molecule. It should be understood that a DNA sequence partially modified by, for example, the presence of plural codons encoding the same amino acid residue due to the degeneracy of codon; and various artificial treatments such as site-specific mutation, random mutation by treating with a mutagen, mutation, deletion, linkage and the like of the DNA fragment by cleaving with a restriction enzyme are included within the present invention as long as it is hybridizable with the DNA represented by SEQ ID No. 1 under stringent conditions and encodes a cellular adhesion molecule even if their sequences are different from the DNA sequence represented by SEQ ID No. 1.

The DNA mutant is acceptable as long as it has a homology with the DNA sequence represented by SEQ ID No. 1 of at least 70%, preferably at least 80%, more preferably at least 90%. The homology in DNA sequence can be analyzed by BLAST (J. Mol. Evol., Vol. 36, pp. 290-300 (1993) ; J. Mol. Biol., Vol. 215, pp. 403-410 (1990)). The term "hybridizable" means that a DNA is hybridizable with the nucleic acid represented by SEQ ID No. 1 by southern hybridization under stringent conditions. For example, if a probe labeled with DIG Labeling kit (Cat No . 1175033 of Rosche Diagnostics) is used , the hybridization is conducted in a DIG Easy Hyb solution (Cat No. 1603558 of Rosche Diagnostics) at the temperature of, for example, 32°C (preferably 37°C, more preferably 42°C) and the membrane is washed in, for example, 0.5×SSC solution (containing 0.1% (w/w) SDS) at 50°C (preferably 65°C) (note: 1 x SSC is 0.15M NaCl and 0.015M sodium citrate) .

The DNA comprising the nucleotide sequence represented by SEQ ID NO:1 or its partial fragment is believed to be useful as a specific probe for diseases in which the protein of the present invention participates such as autoimmune disease, immunodeficiency, allergic disease, inflammatory disease, tumor and the like.

The DNA of the present invention can be used to commercially produce HRC12337. And, the DNA can be used for testing the expression status of the protein of the present invention in a tissue by labeling with an enzyme or the like. That is, an expression amount of mRNA as an index of an expression amount of the protein of the present invention in a cell is confirmed by using the DNA as a probe so that a cell and culturing conditions of the cell suitable for the preparation of the protein of the present invention can be determined. In addition, diseases in which the protein of the present invention participates, especially autoimmune disease, immunodeficiency, allergic disease, inflammatory disease, tumor and the like can be diagnosed.

Further, an abnormality or polymorphism on the nucleic acid sequence can be tested and/or diagnosed by any method such as PCR-RFLP (Restriction fragment length polymorphism) method, PCR-SSCP (Single strand conformation polymorphism) method, sequencing method and the like, using a part of the DNA of the present invention as a primer.

And, the DNA of the present invention can be used in gene therapy for preventing or treating diseases such as autoimmune disease, immunodeficiency, allergic disease, inflammatory disease, tumor or the like by introducing the DNA of the present invention into an *in vivo* cells.

The DNA of the present invention is very useful in the production of a transformant, the preparation of a recombinant protein HRC12337 using said transformant and the screening of a compound specifically inhibiting the expression of HRC12337 .

The transformant of the present invention can be produced according to a method known for those skilled in the art. For example, the DNA of the present invention can be incorporated into a suitable host cell using any one of vectors commercially available or easily obtained by those skilled in the art. Then, the expression of the gene hrc12337 within the host cell can be suitably controlled by placing the gene hrc12337 under the influence of an expression control gene, typical examples of which are a promoter and an enhancer. This technique is suitable for being used in the production of HRC12337 using the transformed host cell as well as the investigation of mechanisms how to regulate the expression of the gene hrc12337 and the screening of an agent capable of controling the expression of the gene.

For example, by contacting any test substances with a cell transformed with the vector including the gene hrc12337 under suitable conditions, an agent capable of enhancing or inhibiting the expression of the gene hrc12337 can be searched among the test substances or evaluated.

By using the DNA of the present invention in combination with a known technique, a transgenic animal can be produced from a suitable animal such as mouse or the like. Further, it is possible to produce the so-called knockout non-human animal in which a orthologue gene corresponding to the human hrc12337 is destroyed if the gene hrc12337 of the present invention is used. By analyzing physiological, biological, pathological and genetic characteristics of this model animal, functions of the gene and the protein of the present invention can be elucidated. Further, by transducing the human hrc12337 of the present invention into an animal in which endogenous genes are destroyed, a model animal having only human hrc12337 can be produced. This model animal is useful in the development and the evaluation of medicines targeting the human hrc12337 transduced.

<HRC12337 protein>

The HRC12337 protein encoded by hrc12337 is a cellular adhesion molecule comprising the amino acid sequence represented by SEQ ID NO:2. Especially, this protein is judged to be a novel cellular adhesion molecule belonging to the sialoadhesin family, from structural characteristics found in its amino acid sequence.

HRC12337 has 2 immunoglobulin domains and a sialic acid binding motif within its molecule and it is homologous with CD33 and AIRM1 at an amino acid level of about 40%. And, an arginine residue in a V domain essential for a sialic acid dependent bonding was conserved (see Figs. 2 and 3) . Therefore, it is understood that the cellular adhesion molecule of the present invention is a protein having an activity of binding to a sialic acid-containing sugar chain similar to other molecules belonging to the sialoadhesin family. Further, it is understood that the cellular adhesion molecule of the present invention expresses specifically on activated lymphocytes and therefore it is a protein having an activity of controlling the recognization between immunocompetent cells and regulating an immune response. An activity of HRC12337 which is the cellular adhesion molecule of the present invention in the binding to a sialic acid-containing sugar chain can be confirmed by, for example, the method shown in Example 5. A specificity of a sialic acid-containing sugar chain recognized by HRC12337 can be determined according to the method described in Curr. Biol., Vol. 4, pp. 965-972 (1994). An activity of HRC12337 which is the cellular adhesion molecule of the present invention in the regulation of an immune response can be confirmed by, for example, a mixed lymphocyte reaction (Procedures in Immunological Experiments II, pp. 738-742, edited by Shunsuke MIGITA, published by Nankodo (1995)). More specifically, an activity of HRC12337 in the regulation of an immune response can be detected as an activity of proliferating lymphocytes and/or an activity of enhancing the production of IL-2 in a mixed lymphocyte reaction, as shown in Example 10. In more detail, the characteristic of HRC12337 is that it enhances the production of IL-2 and it does not affect the production of TNF$\alpha$ and the production of IL-8. An activity of proliferating lymphocytes and/or an activity of enhancing the production of IL-2 means that a determined value about the proliferation of lymphocytes and/or the production of IL-2 is different between the presence and absence of HRC12337 protein in a mixed lymphocyte reaction. For example, the difference in terms of a variation of determined value calculated by the following equation is 10% or higher, preferably 30% or higher, more preferably 50% or higher, even preferably 70% or higher, especially preferably 90% or higher.

variation of determined value (%) =

[absolute value of (determined value in the presence of HRC12337

protein) minus (determined value in the absence of HRC12337

protein)] / (determined value in the presence of HRC12337

protein) * 100

The above determined value is suitably determined depending on the kind of a system capable of confirming the activityoftheprotein. For example, if a system for determining the production of IL-2 in a mixed lymphocyte reaction as shown in Example 10 is used, an amount (pg/ml) of IL-2 produced can be determined. When the determined value in the presence of HRC12337 protein is higher than that in the absence of HRC12337 protein, a test substance can be judged to have an activity of enhancing the production of IL-2. If a system for determining the proliferation of lymphocytes in a mixed lymphocyte reaction as shown in Example 10 is used, an absorbance can be determined since an amount of a cellular DNA incorporated by bromodeoxyuridine is determined by an ELISA method. When the determined value in the presence of HRC12337 protein is higher than that in the absence of HRC12337 protein, a test substance can be judged to have an activity of proliferating lymphocytes. Of course, if values from background and/or noises is contained in a determination system, they should be subtracted.

HRC12337 has not an inhibitory signal motif ITIM (immunoreceptor tyrosine-based inhibitory motif) unlike to CD33 and AIRM1. And, the expression of HRC12337 is induced by peripheral leukocytes activated with PHA (phytohemagglutinin). Moderate expression is observed in spleen, thymus, uterus and testis. The expression in other organs is very small. This is very characteristic. And, as shown in Example 8 determining the concentration of the HRC12337 protein in sera of various patients, patients suffering frompollinosis, atopic dermatitis and angiitis tend to show higher value than that of a healthy subject. Thereby, it became clear that HRC12337 are associated with the above diseases.

As described in the above, HRC12337 sufficiently keeps characteristics which are found in the sialoadhesin family at a domain structure level. While, it has such characteristics that it has not ITIM, that its expression is induced upon the stimulation of peripheral leukocytes, that it shows an activity of proliferating lymphocyte in a mixed lymphocyte reaction, that it enhances the production of IL-2 in a mixed lymphocyte reaction, that it shows higher value in sera of patients suffering from pollinosis, atopic dermatitis and angiitis and the like. From these facts, it is strongly guessed that HRC12337 has a characteristic role which is not found in other molecules belonging to the sialoadhesine family with respect to the regulation of immune functions. Therefore, it is expected that a pharmaceutical compound targeting HRC12337 may be useful as a medicine having unknown characteristic.

A polypeptide or a protein comprising an amino acid sequence wherein substitution, deletion and/or addition of one or more amino acids has occurred in an amino acid sequence represented by SEQ ID NO:2 of the protein is included within the scope of the present invention as long as it is a cellular adhesion molecule.

Side chains of amino acid residues which are constitutional elements of a protein are different in terms of hydrophobicity, charge, size and the like, but they are known to have several highly conservative relationships since they do not substantially affect a three-dimensional structure (also called as configuration) of the entire protein. Examples of the substitutions of amino acid residues include glycine (Gly) and proline (Pro); Gly and alanine (Ala) or valine (Val); leucine (Leu) and isoleucine (Ile); glutamic acid (Glu) and glutamine (Gln) ; aspartic acid (Asp) and asparagine (Asn) ; cysteine (Cys) and threonine (Thr) ; Thr and serine (Ser) or Ala; lysine (Lys) and arginine (Arg); and the like. Since Ala, Val, Leu, Ile, Pro, methionine (Met), phenylalanine (Phe), tryptophane (Trp), Gly and Cys are classified as nonpolar amino acids, they are understood to have similar properties to each other. Non-charged polar amino acids include Ser, Thr, tyrosine (Tyr), Asn and Gln. Acidic amino acids include Asp and Glu. Basic amino acids include Lys, Arg and histidine (His). Even if the conservation as defined above is lost, many mutants maintaining functions essential for the protein (in the present invention, the function as a cellular adhesion molecule) are known for those skilled in the art. Further, in several similar proteins conserved between different species, it is recognized that they maintain essential functions even if several amino acids are deleted or inserted concentratedly or scatteringly.

Accordingly, a mutant protein resulting from substitution, insertion, deletion and/or addition of one or more amino acids in the amino acid sequence represented by SEQ IDNo. 2 are included within the scope of the present invention as long as it is a cellular adhesion molecule, i.e. it has function similar to those of the HRC12337 protein of the present invention. Having similar function means that the mutant maintain at least one activity selected from an activity of binding to sugar chain, an activity of proliferating lymphocytes and an activity of enhancing the production of IL-2.

The above changes in amino acids are found in the nature such as the diversity caused by a gene polymorphism or the like. Further, it can be produced artificially according to a known method for those skilled in the art, for example, mutagenesis using a mutagene such as NTG and site-directed mutagenesis using various recombinant gene techniques. The site and the number of the mutation of amino acids are not particularly limited as long as the resultant mutant protein is a cellular adhesion molecule. The mutation number is generally within several tens of amino acids, preferably within 10 amino acids, more preferably within 1 or several amino acids.

In the present invention, HRC12337 can be understood as an entire molecule having all domain structures as described above. Alternatively, it can be understood as a partial peptide maintaining characteristic domains, especially domains participating in a ligand bindability. It has been reported that among transmembrane proteins, a partial fragment having a ligand binding site may present as a free (or solubilized) partial peptide, for example, by separating it fromother domains while maintaining its characteristic configuration. Since such a partial peptide maintains a bindability to a specific ligand, it can be possible to search compounds having a bindability to said protein using the above partial peptide. It should be understood that a partial peptide of HRC12337 is a substance substantially equivalent to the protein of the present invention as long as it has a ligand bindability. It can be understood that the ligand for HRC12337 is a sialic acid-containing sugar chain. From facts that HRC12337 is present *in vivo* as a soluble type and that soluble HRC12337-Fc shows an activity of enhancing the proliferation of lymphocytes and the production of IL-2, it is thought that soluble HRC12337 per se may function as a ligand and bind to any receptor so that an activity of regulating an immune response may be exhibited. It is estimated that in HRC12337, a ligand bindability or an activity of regulating an immune response is kept in its partial peptide at N-terminal containing an extracellular region. Therefore, a protein containing an extracellular region of HRC12337 and a DNA encoding said protein are included within the scope of the present invention. That is, the present invention includes the following (1) to (4):

(1) the following DNA (a) or (b), i.e.

(a) a DNA comprising the nucleotide sequence represented by SEQ ID NO:4 or
(b) a DNA hybridizable with the DNA of SEQ ID NO:4 under stringent conditions and encoding a protein which has at least one activity selected from an activity of binding to a sialic acid-containing sugar chain, an activity of proliferating lymphocyte and an activity of enhancing the production of IL-2;

(2) a protein encoded by the DNA as defined in (1);
(3) the following DNA (a) or (b), i.e.

(a) a protein comprising the amino acid sequence represented by SEQ ID NO:5 or
(b) a protein comprising an amino acid sequence wherein deletion, substitution or addition of one or more amino acids has occurred in an amino acid sequence of SEQ ID NO: 5 and having at least one activity selected from an activity of binding to a sialic acid-containing sugar chain, an activity of proliferating lymphocyte and an activity of enhancing the production of IL-2;

(4) a fused protein comprising the protein as defined in (3). Other preferable embodiment of the partial peptide includes a peptide containing either Ig domain I (57 to 144 a.a. in SEQ ID NO:2) or Ig domain II (187 to 239 a.a. in SEQ ID NO:2) . In view of a ligand bindability, a partial peptide containing at least Ig domain I is preferable. A partial peptide to which all or a part of other domains are linked or a fused protein with any other protein or peptide is possible as long as it contains at least Ig domain I (57 to 144 a.a. in SEQ ID NO:2). Other polypeptide linked to an extracellular region of HRC12337 is not especially limited as long as the resultant fused protein has at least one activity selected from an activity of binding to a sialic acid-containing sugar chain as the extracellular region of HRC12337, an activity of proliferating lymphocyte and an activity of enhancing the production of IL-2. One example of the preferable fused protein is HRC12337-His represented by SEQ ID NO:6 or HRC12337-Fc SEQ ID NO:7. In case of a protein having a signal sequence, the protein from which the signal sequence is deleted may function as amature protein. Therefore, it is understood that a mature peptide prepared by deleting a signal sequence from the protein of the present invention is a substance substantially equivalent to the present protein. In case of HRC12337, it is expected that a signal sequence is present near amino acid residues of amino acid Nos. 7 to 20 of the amino acid sequence represented by SEQ ID NO:2.

The protein of the present invention or its partial peptide can be used in screening an agent capable of regulating an activity of said protein. The thus-screened compounds and the like are expected to be useful as an effective therapeutic or preventive agent for diseases associated with the protein of the present invention such as autoimmune disease, immunodeficiency, allergic disease, inflammatory disease, tumor and the like.

<Antibody>

Further, the present invention provides an antibody binding to HRC12337. The antibody of the present invention is an antibody specifically recognizing the entire HRC12337 or its partial peptide as an antigen. It includes amonoclonal antibody and/or a polyclonal antibody. And, it may be an antibody belonging to any one of five classes (IgG, IgA, IgM, IgD and IgE) classified by the structure, physical-chemical properties and immunological properties of immunoglobulins or either subclass classified by the type of H chain. Further, it may be a fragment such as $F(ab')_2$ produced by digesting an immunoglobulin with, for example, pepsin, Fab produced by digesting an immunoglobulinwithpapain and the like, or a chimera antibody and a humanized antibody. In addition, an antibody having functions of not also specifically recognizing HRC12337 or its partial peptide but also regulating an activity of HRC12337 is also included within the scope of the present invention. Example of an antibody having a function of regulating an activity of HRC12337 includes a neutralizing antibody inhibiting the binding of HRC12337 to a ligand. These antibodies are useful in investigating or clinical detection of HRC12337 and the like.

<Antisense nucleic acid>

The present invention provides the so-called antisense nucleic acid capable of inhibiting the biosynthesis of HRC12337 at a nucleic acid level *in vivo.* The antisense nucleic acid means a nucleic acid which binds to DNA or RNA involved in carrying a genetic information during either of a transcription stage from a genome region to a pre-mRNA essential for the production of mRNA encoding HRC12337, a processing stage from the pre-mRNA to a mature mRNA, a stage of passing through a nuclear membrane or a translation stage into a protein so as to affect the normal stream of the transmission of the genetic information and thereby to inhibit the expression of the protein. It may comprises a sequence complementary to the entire or either part of the nucleic acid sequence of the gene HRC12337. Preferably, it is a nucleic acid (including DNA and RNA) comprising a sequence corresponding to or complementary to the nucleic acid sequence represented by SEQ ID NO: 1 or 3. When the mRNA transcripted from the genome region contains an intron structure or a untranslated region at 5' or 3'-terminal, an antisense nucleic acid corresponding to or complementary to the sequence of the untranslated region will have functions equivalent to those of the antisense nucleic acid of the present invention.

The antisense nucleic acid of the present invention includes a DNA and an RNA as well as all of derivatives similar to the DNA and the RNA in configuration and functions. Example of the antisense nucleic acid includes a nucleic acid having any other substance bound at 3'- or 5'-terminal, a nucleic acid wherein at least one of bases, sugars and phosphates of the oligonucleotide is substituted or modified, anucleic acid having a non-naturally occurring base, sugar or phosphate, a nucleic acid having a backbone other than the sugar-phosphate backbone and the like. These nucleic acids are suitable as derivatives, in which at least one of a nuclease resistance, a tissue selectivity, a cell permeability and a binding power is improved. That is, the form of the nucleic acid is not limited as long as the nucleic acid can inhibit the activity and the expression of HRC12337.

And, the antisense nucleic acid having a nucleotide sequence complementary to the nucleotide sequence hybridizable with a loop portion of mRNA forming a stem loop, i.e. the nucleotide sequence of a region forming a stem loop is generally preferable in the present invention. Alternatively, an antisense nucleic acid capable of binding to near a translation initiation codon, a ribosome binding site, a capping site and a splicing site, i.e. an antisense nucleic acid having a sequence complementary to that of these sites is also preferable since generally it can be expected to be very effective in inhibiting the expression.

In order to make the above antisense nucleic acid introduced into a cell and act efficiently, it is preferable that the length of the antisense nucleic acid of the present invention is 15 to 30 bases, preferably 15 to 25 bases, more preferably 18 to 22 bases.

The effect of the antisense nucleic acid of the present invention in the inhibition of the expression can be evaluated by a known method, for example, by preparing an expression plasmid by linking a reporter gene such as luciferase and the like to the DNA containing a part of an expression control region, a 5'-untranslated region, a region near a translational initiation site or a translational region of the gene of the present invention, adding a test substance in a system such as a system comprising in vitro transcription (Ribo max systems; Promega) combined with *in vitro* translation (Rabbit Reticulocyte Lysate Systems; Promega) under the condition where the gene of the present invention is transcripted or translated and determining an expression amount of the reporter gene.

The antisense nucleic acid of the present invention is useful as a preventive or therapeutic agent for diseases associated with HRC12337 since it can inhibit the expression of hrc12337 *in vivo.*

## DETAILED DESCRIPTION OF THE INVENTION

<Nucleic acid>

Example of the method for obtaining the DNA of the present invention from a DNA library includes a method comprising screening a suitable genomic DNA library or cDNA library according to a screening method such as a screening method via hybridization, an immunoscreening method using an antibody and the like, amplifying a clone having the desired DNA and digesting the DNA with a restriction enzyme or the like. In the screening method via hybridization, the hybridization can be conducted for any cDNA library using the DNA having the nucleotide sequence represented by SEQ ID No. 1 or a part thereof labeled with $^{32}P$ or the like as a probe according to a known method (see, for example, Maniatis, T. et al., Molecular Cloning, a Laboratory Manual, Cold Spring Harbor Laboratory, New York (1982)) . The antibody used in the immunoscreening method may be the antibody of the present invention as described below. The novel DNA of the present invention may be also obtained by PCR (Polymerase Chain Reaction) using a genomic DNA library or a cDNA library as a template. PCR is conducted for any DNA library according to a known method (see, for example, Michael, A.I. et al., PCR Protocols, a Guide to Methods and Applications, Academic Press (1990)) using sense and antisense primers prepared based on the nucleotide sequence of SEQ IDNO:1, thereby the DNA of the present invention can be obtained. As the DNA library used in the above methods, a DNA library having the DNA of the present invention is selected and used. Any DNA library can be used as long as it comprises the DNA of the present invention. A commercially available DNA library may be also used. Alternatively, a cDNA library may be constructed according to a known method (see J. Sambrook et al., Molecular Cloning, a Laboratory Manual, 2nd ed., Cold Spring Harbor Laboratory, New York (1989)) by selecting cells suitable for the construction of the cDNA library from cells having the DNA of the present invention.

And, the DNA of the present invention can be prepared based on the sequence as disclosed herein by a chemical synthetic technique such as a phosphoramidite method and the like.

The recombinant vector including the DNA of the present invention may have any form such as a cyclic form or a linear form. The recombinant vector may have any other nucleotide sequence in addition to the entire or a part of the DNA of the present invention, if necessary. "Apart" means, for example, a DNA encoding a partial peptide of the protein of the present invention. The other nucleotide sequence includes an enhancer sequence, a promoter sequence, a ribosome binding sequence, a nucleotide sequence used for amplifying the number of copies, a nucleotide sequence encoding a signal peptide, a nucleotide sequence encoding other polypeptide, a polyA addition sequence, a splicing sequence, a replication origin, a nucleotide sequence of the gene acting as a selective marker and the like. One preferable example of the recombinant vector of the present invention is an expression vector.

In the gene recombination, it is possible to add a translational initiation codon or a translational stop codon to the DNA of the present invention using a suitable synthetic DNAadaptor, and to newly produce or delete a suitable restriction site within the nucleotide sequence. This is the technique routinely conducted by those skilled in the art. Such a processing can be suitably and easily conducted based on the DNA of the present invention.

As the vector including the DNA of the present invention, a suitable vector is selected and used depending on the type of a host used. The vector may be a plasmid. Alternatively, various viruses may be used, non-limiting examples of which include bacteriophage, baculovirus, retrovirus, vaccinia virus and the like.

The gene of the present invention can be expressed under the control of a promoter sequence inherent in said gene. Using the expression system, an agent promoting or inhibiting the transcription of the gene of the present invention can be efficiently searched. Any other suitable expression promoter can be used by linking it to the promoter sequence inherent in said gene upstream of the gene of the present invention or replacing it with the promoter sequence. In this case, the promoter may be selected depending on a host or an object of expression. For example, if a host is E. coli, a T7 promoter, a lac promoter, a trp promoter, a $\lambda$PL promoter or the like can be used. If a host is a yeast, a PHO5 promoter, a GAP promoter, an ADH promoter or the like can be used. If a host is an animal cell, a promoter from SV 40, a retro virus promoter, an elongation factor $\alpha$ promoter or the like can be used. These lists are not exclusive.

A method for introducing the DNA into a vector is known (see J. Sambrook et al., Molecular Cloning, a Laboratory Manual, 2nd ed., Cold Spring Harbor Laboratory, New York (1989)). That is, each of the DNA and the vector is digested with a suitable restriction enzyme and the resultant fragments are ligated with a DNA ligase.

<Protein>

The protein of the present invention can be prepared from various cells and tissues expressing said protein. Alternatively, it can be chemically synthesized in a peptide synthesizer (for example, Peptide Synthesizer Model 433A; Applied Biosystems Japan) or it can be produced by recombination method using a suitable host cell selected fromprokaryotic cells and eukaryotic cells. However, a genetic engineering technique and a recombinant protein produced thereby are preferable in view of purity.

A host cell to be transformed with the recombinant vector described in the previous section is not limitative. Many cells such as cells of low organisms available in genetic engineering techniques, typical examples of which are E. coli, B. subtilis and S.cerevisiae; and animal cells, typical examples of which are insect cell, COS7 cell, CHO cell and HeLa cell, can be used in the present invention.

The transformant of the present invention can be obtained by transforming a suitable host cell with the recombinant vector of the present invention. As the method of introducing the recombinant vector described in the previous section into a host cell, some methods are known such as an electroporation, a protoplast method, an alkali metal method, a calcium phosphate precipitation method, a DEAE dextran method, a microinjection method, a method using virus particles and the like (see "Handbook of Genetic Engineering", special Issue of Experimental Medicines, published by Yodosha Co., Ltd. (March 20, 1991)). Either method may be used.

For preparing the present protein by a genetic engineering technique, the above transformant is cultured to obtain a culture mixture followed by purifying the protein. The transformant can be cultured according to a standard method. Many textbooks are available, for example, "Experimental Procedures in Microbiology", edited by The Japanese Biochemical Society, published by Tokyo Kagaku Dozin Co., Ltd. (1992)) describing the culture of transformants, for reference.

As a method for purifying the protein of the present invention from the culture mixture, a suitable method is selected among conventional methods for purifying proteins. The conventional methods include salting out, ultrafiltration, isoelectric precipitation, gel filtration, electrophoresis, various affinity chromatographies including ion-exchange chromatography, hydrophobic chromatography and antibody chromatography, chromatofocusing, adsorption chromatography, reverse phase chromatography and the like. If necessary, HPLC systems or the like may be used to conduct several purification methods in a suitable order.

It is possible to express the protein of the present invention as a fused protein with any other protein or tag such as glutathione S transferase, ProteinA, hexahistidine tag, FLAG tag and the like. The thus-expressed fused protein may be separatedwith a suitable protease such as thrombin, enterokinase and the like. This may be more effective for the preparation of the protein. For purifying the protein of the present invention, conventional methods may be suitably combined. Especially if the protein is expressed in the form of a fused protein, it is preferable to purify according to a method characteristic to such a form.

One of methods for preparing the present protein by a genetic engineering technique is the synthesis of a cell-free system using a recombinant DNA molecule (J. Sambrook et al., Molecular Cloning, 2nd ed. (1989)).

As mentioned above, the protein of the present invention can be prepared in the form of a single protein or a fused protein with any other different protein. The form of the protein of the present invention is not limited to them. Further, it is possible to transform the protein of the present invention to various forms. For example, it is thought that the protein can be processed according to various methods known for those skilled in the art such as various chemical modifications on the protein, the binding of the protein to a polymeric substance such as polyethylene glycol and the like, the binding of the protein to an insoluble carrier and the like. And, the presence or absence of glycosylation or the difference in glycosylation degree is found depending on a host cell used. It should be understood that all of the above proteins are included within the scope of the present invention as long as said proteins function as cellular adhesion molecules.

The protein of the present invention is useful since it can be used as an antigen for the preparation of an antibody or it can be used for the screening of an agent capable of binding to said protein or an agent capable of regulating an activity of said protein.

HRC12337 of the present invention can express a desired molecule on a surface of the above transformant, especially an animal cell at a high level by culturing the cell. When a suitable fragment of HRC12337 such as an extracellular region protein fragment thereof is prepared as a soluble protein, such a fragment can be prepared by preparing a transformant using a DNA encoding the extracellular region or each domain according to the above method and culturing the resultant transformant so as to secret it in the culture supernatant.

On the other hand, when HRC12337 is present in a periolasm or cytoplasm of a transformant, the transformant suspended in a suitable buffer is treated by an ultrasonic treatment, a freeze-thawing treatment, a treatment with lysozyme or the like to destroy a cell wall and/or a cell membrane and further treated by a centrifugation, a filtration or the like to obtain amembrane fraction containing the protein of the present invention. This fraction is solubilized using a suitable surfactant to prepare a crude solution. Thereafter the desired protein can be isolated and purified from the crude solution according to a routine method.

<hrc12337 gene recombinant non-human animal>

The present invention provides a recombinant non-human animal of hrc12337 gene. The recombinant non-human animal of hrc12337 gene includes transgenic non-human animals and knock out non-human animals. In the hrc12337 gene transferred non-human animal, the expression of the protein of the present invention can be controlled in terms of its level, its time, its sites and the like since the gene encoding said protein is artificially inserted on a chromosome of a non-human animal. Non-limiting examples of anon-human animal include cattle, goat, sheep, pig, mouse, horse, chicken and the like. Among the non-human animals, a non-human mammalian animal is preferable.

By using the gene hrc12337 of the present invention, a transgenic non-human mammalian animal can be produced. The transgenic non-human mammalian animal can be produced according to a routine method conventionally used in the production of transgenic animals (see, for example, "Experimental Manual of Genesis, published by Kodansha Scientific Ltd., edited by Motoya KATSUKI under supervision of Tatsuji NOMURA (1987)). That is, the gene or the recombinant vector of the present invention is introduced into a totipotent cell of a non-human animal to produce subjects and thereafter only a subject in which the gene introduced is incorporated in a genome of a somatic cell is selected.

Specifically, in case of a transgenic mouse, a DNA prepared such that the hrc12337 gene can be expressed is directly poured into a pronucleic oosperm obtained from a normal C57Black/6 mouse . More specifically, a construct is prepared by introducing the hrc12337 gene downstream of a suitable promoter by linking. Thereafter, a linear DNA is obtained by removing the sequence from a prokaryote as much as possible, if necessary. This DNA is directly poured into a pronucleus of the pronucleic oosperm using a fine glass needle.

The oosperm is transplanted in an uterus of another pseudopregnant mouse as an allomother. The pseudopregnant mouse is generally prepared by mating an ICR female mouse with a vasectomized or vasoligated male mouse. A genomic DNA is extracted from a tissue from the transplated embryo and confirmed whether or not the hrc12337 gene is introduced by PCR or southern blotting, thereby a transgenic mouse is obtained.

The so-called "knockout mouse" can be produced based on the nucleotide sequence of hrc12337 (or a mouse homologous gene of hrc12337). The term "knockout mouse" used herein means a mouse in which an endogenous gene encoding the protein of the present invention is knocked out (inactivated). The knockout mouse can be produced by, for example, a positive-negative selection method via homologous recombination (see, for example, US patent Nos. 5, 464, 764, 5, 487, 992 and 5, 627, 059; Proc. Natl. Acad. Sci. USA, Vol. 86, pp. 8932-8935 (1989); Nature, Vol. 342, pp. 435-438 (1989)). Such a knockout mouse is one embodiment of the present invention.

Recently, the production of clone animals by nuclear transplantation in medium or large animals became possible. In this connection, transgenic and knockout animals have been practically produced using this technique. That is, a somatic cell or a germinal cell is subjected to homologous recombination based on the nucleotide sequence of hrc12337 (or a homologous gene of hrc12337 in each animal) in the same way as that applied to ES cells and then a nucleus is obtained from the resultant cell and used to obtain a clone animal. This animal is a knockout animal in which hrc12337 (or a homologous gene of hrc12337 in each animal) is lost. Or, hrc12337 (or a homologous gene of hrc12337 in each animal)is introduced in any cell of any animal and then the resultant nucleus is used to obtain a clone animal, thereby a transgenic animal can be produced. Such a knockout non-human animal and a transgenic non-human animal are one embodiment of the present invention irrespective of its species.

<Antibody>

The antibody of the present invention may be polyclonal or monoclonal. Either antibody can be obtained by referring to a known method (see, for example, "Experimental Procedures in Immunology", editedby Japan Society for Immunology, published by Japan Society for Immunology), as describe below in brief.

For obtaining the novel antibody, an animal is administered with the protein of the present invention as an immunizing antigen and if necessary a suitable adjuvant such as Freund's complete adjuvant (FCA), Freund's incomplete adjuvant (FIA) and the like. If necessary, a booster at an interval of 2 to 4 weeks may be conducted. After the booster, blood sample is collected for the preparation of an anti-serum. The protein of the present invention used as an antigen is that obtained in any method as long as it has a purity sufficient to be usable in the preparation of an antibody. A partial polypeptide of the protein of the present invention may be suitably used as an immunizing antigen. If the polypeptide used as an immunizing antigen is a low-molecular weightpolypeptide, i.e. a polypeptide comprising about 10 to 20 amino acids, it may be linked to a carrier such as keyhole limpet hemocyanin (KLH) and the like and used as an antigen. Animals to be immunized include those conventionally used in immunological experiments by those skilled in the art such as rat, mouse, rabbit, sheep, horse, chicken, goat, pig, cattle and the like, among which preferably a species capable of producing the desired antibody is selected and used. However, it is not limited thereto.

A polyclonal antibody can be obtained by purifying the resultant anti-serum. The purification may be conducted by suitably combining known methods such as salting-out, ion exchange chromatography, affinity chromatography and the like.

A monoclonal antibody is obtained as follows: An antibody-producing cell such as a spleen cell, a lymphocyte and the like is taken from an immunized animal. The cell is fused with a myeloma cell or the like according to a known method using polyethylene glycol, Sendai virus, an electric pulse or the like to produce a hybridoma. Thereafter, a clone producing an antibody which binds to the protein of the present invention is selected and cultured. By purifying a supernatant of the culture of the selected clone, amonoclonal antibody is obtained. The purification may be conducted by suitably combining known methods such as salting-out, ion exchange chromatography, affinity chromatography and the like.

And, the novel antibody is also obtained by a genetic engineering technique. For example, a mRNA is obtained from a spleen cell or a lymphocyte of an animal immunized with the protein of the present invention or its partial polypeptide or from a hybridoma producing a monoclonal antibody against the protein of the present invention or its partial polypeptide. Based on the thus-obtained mRNA, a cDNA library is constructed. A clone producing the antibody which reacts with the antigen is screened and the thus-screened clone is cultured. The desired antibody can be purified from the culture mixture by combined knownmethods. When the antibody is used for therapy, a humanized antibody is preferable with respect to immunogenicity. The humanized antibody can be prepared by immunizing a mouse whose immune system has replaced with a human immune system (see, for example, Nat. Genet., Vol. 15, pp. 146-157 (1997)). Alternatively, the humanized antibody can be engineered using hypervariable regions of the monoclonal antibody (Method in Enzymology, Vol. 203, pp. 99-121 (1999)).

<Antisense nucleic acid>

The antisense nucleic acid can be prepared according to a known method (see, for example, edited by Stanley T. Crooke and Bernald Lebleu, in Antisense Research and Applications, published by CRC Publisher, Florida (1993)). If DNA and RNA are native, the antisense nucleic acid of the present invention can be obtained by synthesizing in a chemical synthesizer or conducting PCR using hrc12337 as a template. Alternatively, a part of derivatives such as methyl phosphonate type and phosphorothioate type can be synthesized in a chemical synthesizer (for example, Expedite Model 8909; Applied Biosystems Japan). Then, such a derivative may be synthesized according to a manual attached to the chemical synthesizer and the thus-synthesized product may be purified by HPLC using a reverse phase chromatography or the like, thereby the antisense nucleic acid can be obtained.

When the DNA and the antisense nucleic acid of the present invention is used as a diagnostic probe, they are labeled with aradioisotope, an enzyme, a fluorescent substance, a luminescent substance or the like according to a known method. Subsequently, a DNA or a mRNA is prepared from a specimen according to a known method and it is used as a test substance. This test substance is reacted with the labeled probe and then the reaction is washed to remove the labeled probe unreacted. If the test substance contains the gene hrc12337 or RNA, said antisense nucleic acid binds thereto. The presence or absence of the binding formation can be known by using a luminescence, a fluorescent, a radioactivity or the like from the enzyme, a fluorescent substance or a luminescent substance labeled; or a radioisotope as an index.

When the DNA, the antisense nucleic acid or the recombinant vector of the present invention is used in clinical applications, it is preferable to use those having a purity suitable for the use of a medicine according to any pharmaceutically acceptable method.

The DNA, the antisense nucleic acid or the recombinant vector of the present invention may be used by directly dissolving or suspending in a suitable solvent. Alternatively, it may be used after encapsulating in a liposome or incorporating in a suitable vector. If necessary, it may be used in a suitable dosage form such as injections, tablets, capsules, eye drops, creams, suppositories, spray, poultices in which pharmaceutically acceptable adjuvants are added. Examples of the pharmaceutically acceptable adjuvants are a solvent, a base, astabilizer, a preservative, a solubilizing agent, an excipient, a buffer and the like.

When the DNA, the antisense nucleic acid or the recombinant vector of the present invention is used in the above dosage form, its administration method and its dose can be selected depending on the age and the sex of a patient, the type and the severity of the disease. Thus, it may be administered in an amount suitable to improve pathological conditions by the suitable method selected from oral, inhalation, transdermal, intravaginal, intraarticular, intrarectal, intravenous, local, intramuscular, subcutaneous and intraperitoneal administrations.

<Screening method>

The present invention relates to a method of screening an agent capable of controling the function or the expression of the protein of the present invention, which comprises using the protein of the present invention, a transformant expressing said protein, the DNA of the present invention, a recombinant vector including said DNA, a transformant produced by transformation with said vector or a hrc12337 gene transferred non-human mammalian animal.

More specifically, the screening method includes:

(1) a method of evaluating an activity of the protein of the present invention in the presence /absence of a test substance;

(2) a method of screening an agent capable of controlling the expression of the protein of the present invention by comparing an expression level of the protein or the gene of the present invention in the presence /absence of a test substance; and the like. Example of the method (1) is a method comprising determining an activity of the protein of the present invention in the presence /absence of a test substance in a system as illustrated in Example 5. Example of the method (2) is a method comprising preparing an expression plasmid prepared by linking a reporter gene such as luciferase or the like to the DNA containing an expression control region, a 5'-untranslated region, a region near a translational initiation site or a part of a translation region of the hrc12337 gene and determining an expression amount of the reporter gene under the condition where the gene of the present invention is transcripted or translated in the presence/absence of a test substance using so as to confirm a transcriptional promotion activity or a transcriptional inhibitory activity of the test substance. The screening method of the present invention comprises the steps of contacting a test substance with the protein of the present, a transformant expressing said protein, the DNA of the present invention, a recombinant vector including said DNA, a transformant produced by transformation with said recombinant vector or the hrc12337 gene transferred non-human animal; detecting a difference in an activity of the protein of the present invention or an expression level of the DNA of the present invention between a group with the addition of the test substance and a group without the addition of the test substance; and selecting the test substance showing the difference as an agent capable of regulating an activity of the protein of the present invention or an agent capable of inhibiting the expression of the DNA of the present invention.

An agent capable of regulating an activity of the protein of the present invention may be an agent capable of imitating(mimic), enhancing(agonist) or inhibiting (antagonist) an activity of the HRC12337 protein. An antagonist is preferable. An agent capable of controling the expression of the DNA of the present invention may be an agent capable of either promoting or inhibiting the expression of the gene hrc12337. An agent capable of inhibiting the expression is preferable. For confirming whether a test substance controls the activity of the protein of the present invention or regulates the expression of the DNA of the present invention, a difference in the activity of the protein or the expression level of the DNA is determined between the addition and no addition of a test substance in a system capable of confirming an activity of the protein or a system capable of confirming the expression of the DNA. The expression level of the DNA may be determined on the basis of an expression strength of the hrc12337 gene into mRNA or the protein. Instead of the expression level of the hrc12337 gene or the HRC12337 protein per se, an expression level of a reporter gene may be detected. The reporter-assay system means an assay method in which an expression amount of a reporter gene arranged downstream of a transcriptional control region is determined so as to screen an agent affecting the transcriptional control region. Examples of the transcriptional control region include a promoter, an enhancer, a CAAT box, a TATA box and the like generally found in a promoter region. As a reporter gene, a CAT (chloramphenicol acetyl transferase) gene, a luciferase gene, a β-galactosidase gene and the like can be used. The expression control region and the 5'-untranslated region of the gene of the present invention can be obtained according to a known method (see "New Experimental Protocol in Cell Engineering", published by Shojunsha Co., Ltd. (1993)). Having function of inhibiting (or suppressing) or enhancing (or promoting) means that a determined value as to the activity of the protein or the expression level of the DNA is different between a group with the addition of a test substance and a group without the addition of a test substance. For example, the inhibition (or suppression) or the enhancement (or promotion) ratio calculated by the following equation is 10% or higher, preferably 30% or higher, more preferably 50% or higher, even preferably 70% or higher, especially preferably 90% or higher.

inhibition (or suppression) or enhancement (or promotion) ratio

(%) =

[an absolute value of (determined value of a group without the

addition of a candidate) minus (determined value of a group with

the addition of a candidate)] / (determined value of a group

without the addition of a candidate) * 100

Either inhibition or enhancement is suitably determined depending on the kind of a system capable of confirming an activity of the protein or a system capable of confirming the expression of the DNA. The determined value is the same. For example, if a system capable of confirming an activity of the protein is a system of determining an activity of binding to a sialic acid-containing sugar chain as shown in Example 5, the formation level of rosettes can be determined. When the determined value in a group with the addition of a test substance is higher than that in a group without the addition of a test substance, the test substance can be judged to have a function of inhibiting an activity of the HRC12337 protein. If a system capable of confirming an activity of the protein is a system of determining the production of IL-2 in a mixed lymphocyte reaction as shown in Example 10, an amount of IL-2 production can be determined. When the determined value in a group with the addition of a test substance is lower than that in a group without the addition of a test substance, the test substance can be judged to have a function of inhibiting an activity of the HRC12337 protein. Of course, if values from background and/or noises is contained in a determination system, they should be subtracted.

Since the protein of the present invention is a cellular adhesion molecule, compounds obtained through the search using the screening method or the transgenic animal described above are expected to be effective therapeutic or preventive agents for diseases such as autoimmune disease, immunodeficiency, allergic disease, inflammatory disease and the like. Non-limiting examples of a test substance include proteins, peptides, oligonucleotides, synthetic compounds, naturally occurring compounds, fermented products, cell extracts, plant extracts, animal tissue extracts and the like. The test substance may be either new or known.

<Assay method, reagent and kit using the present antibody>

The present invention provides

(1) an assay method for the HRC12337 protein in a test sample which comprises using the antibody of the present invention;
(2) an assay reagent or kit for the HRC12337 protein in a test sample which comprises containing the antibody of the present invention ;
(3) an assay method for the HRC12337 protein as mentioned in

(1) which comprises determining the increase or decrease of an amount of the HRC12337 protein in a human body fluid to predict, detect or diagnose a dysfunction of HRC12337, diseases accompanied with the dysfunction or conditions associated with the diseases; and
(4) an assay method as mentioned in (3) wherein the disease is at least one selected from pollenosis, atopic dermatitis and angiitis.

The assay method of the present invention comprises a step using the antibody of the present invention. Preferably this step is a step comprising trapping a test substance, i.e. the HRC12337 protein, in a test sample by an antigen-antibody reaction of said test substance with the antibody of the present invention. Principle of detecting a test substance by the assay method of the present invention are not particularly limited, examples of which include an agglutination method, a sandwich method, a solid phase direct method, a solid phase binding method, a competitive method and the like. Among them, a sandwich method and a competitive method are preferable, a sandwich method being especially preferable.

In an agglutination method, an antibody binds to surfaces of particles such as latex particles and erythrocytes (for example, sheep erythrocytes) such that the particles are agglutinated if the HRC12337 protein is present. In this method, the HRC12337 protein is determined in terms of an agglutination degree of the particles. In this agglutination method, conventionally used particles such as gelatin, microbeads, carbon particles and the like can be used in place of latex particles and erythrocytes.

In a sandwich method, a solid phase direct method, a solid phase binding method and a competitive method, a labeled antibody or antigen is used. The determination can be conducted according to the principles of enzyme immunoassay (EIA) , radioimmunoassay (RIA), chemiluminescence immunoassay, fluoroimmunoassay, time-resolved fluoroimmunoassay (TR-FIA), immunochromatography assay and the like.

A sandwich method, a solid phase direct method and a competitive method based on the principle of EIA which is one of the preferable embodiment of the assay method of the present invention will be described below.

In a sandwich method via EIA, an antibody or a secondary antibody which recognizes the HRC12337 protein and is labeled with an enzyme such as peroxidase, alkaline phosphatase, β-galactosidase or the like is provided. Especially a polymerized peroxidase-labeled antibody is preferable. And, an antibody recognizing the HRC12337 protein is adsorbed on a solid phase to be used. After a sample or a standard is added to the solid phase, the above enzyme-labeled antibody is added so as to conduct an antigen-antibody reaction. Excess enzyme-labeled antibody is removed by washing. Thereafter a chromophoric substrate selected depending on an enzyme used, for example o-phenylenediamine and $H_2O_2$, p-nitrophenyl phosphoric acid, 2-nitrophenyl-β-D-galactoside or the like is added to react with the enzyme. Since the substrate is colored depending on an amount of the enzyme and thereby an amount of the HRC12337 protein in a sample, the concentration of the HRC12337 protein can be quantified by determining an amount of the resultant colored product.

In a solid phase direct method, a sample is directly adsorbed on a solid phase. Surfaces on the solid phase where the HRC12337 protein is not adsorbed are blocked with a protein not affecting its assay system, for example BSA (bovine serum albumin) or the like and then an enzyme-labeled antibody recognizing the HRC12337 protein is added and reacted. The subsequent procedures are similar to those in the above sandwich method to thereby qualitatively or quantitatively determine the HRC12337 protein.

In a competitive method, a predetermined amount of the HRC12337 protein recognized by an antibody used is directly adsorbed on a solid phase. After the solid phase is blocked, an enzyme-labeled antibody recognizing the HRC12337 protein and a sample are added thereto. They are reacted for certain period and the solid phase is washed to remove substances unbound to the solid phase, to which a chromophoric substrate is added to react with the enzyme. After the reaction, the inhibition in binding of the enzyme-labeled antibody to the HRC12337 protein onto the solid phase is determined to thereby quantify the HRC12337 protein in the sample.

The HRC12337 protein in a sample may be quantified by adsorbing an antibody onto a solid phase, adding an enzyme-labeled HRC12337 protein and a sample simultaneously and then determining the inhibition in binding of the enzyme-labeled product to the immobilized antibody due to the addition of the sample.

Any method other than the above-mentioned assay methods includes an assay method comprising conducting an antigen-antibody reaction in a liquid phase, separating the HRC12337 protein bound to a labeled antibody from the HRC12337 protein unbound according to an agglutinating precipitation method using an antibody or a physical-chemical technique and then quantifying. Alternatively, it is possible to determine the HRC12337 protein by preparing a secondary antibody recognizing an antibody which recognize the HRC12337 protein, labeling the secondary antibody and then conducting an antigen-antibody reaction.

In either a sandwich method, a solid direct method or a competitive method, the combination of a labeled enzyme and a chromophoric substrate may be replaced with the combination of a labeled enzyme and a bioluminescent or chemiluminescent substrate or the combination of a labeled enzyme and a fluorescent substrate or the like. Typical examples of the combination of an enzyme and a luminescent substrate include alkaline phosphatase-AMPPD, horseradish peroxidase-luminol, luciferase-luciferin and the like. Examples of the combination of an enzyme and a fluorescent substrate include alkaline phosphatase-umbelliferyl phosphate, horseradish peroxidase-p-hydroxyphenyl propione and the like.

Further, in the above three assay methods, it is possible to quantify the HRC12337 protein in a sample by using an antibody or antigen directly or indirectly labeled with a radioactive substance, a chemiluminescent substance or a fluoroluminescent substance in stead of an enzyme and determining an intensity of radioactivity, luminescence or fluorescence.

As a radioactive substance, $^{125}I$, $^{131}I$ and the like are generally used. Typical chemiluminescent substances include acridium ester and the like. For determining a fluorescent intensity, an assay method comprising directly or indirectly binding a chelating agent to an antibody or an antigen, exposing it to an excitation radiation and determining a fluorescent intensity issued from a rare earth metal bound to the chelating agent with time-resolution so as to quantify the HRC12337 protein in a sample is also useful. This method is more sensitive. Typical examples of the rare earth metal include europium.

The object of the assay method of the present invention is to detect or determine the HRC12337 protein in a sample. A sample to be tested includes a body fluid, a tissue or a cell of an animal (especially human), a body of a bacteria and their extract, culture supernatant, smear and slice. The body fluid is preferable. More preferable sample is selected from blood, plasma, serum, urine, liquor, lymph, saliva, ascites andpleural effusion.

By using the assay method of the present invention, the determination of the HRC12337 protein in body fluids of healthy subjects and patients suffering from different diseases is possible. And, the concentration of the HRC12337 protein in body fluids could first determined by the present invention. In addition, it became clear that the concentration of the HRC12337 protein varies depending on the type of diseases. When the concentration of the HRC12337 protein in body fluids of patients suffering fromdifferent diseases is comparedwith those of healthy subjects, the determined values are statically analyzed according to a method conventionally used by those skilled in the art so as to judge whether or not a difference therebetween is significant.

The assay reagent and the assay kit of the present invention can be constituted in accordance with the above assay method and the like.

## BRIEF DESCRIPTION OF DRAWINGS

Fig. 1 shows the structure of a sialoadhesin family. S-S represents a crosslinking by disulfide bond.

Fig. 2 shows the results of the alignment between HRC12337 and the known sialoadhesin family. In this figure, the left lane shows a protein represented by GenBank accession number. NP_001763 represents a human CD33 antigen. NP_057627 represents a human D-siglec precursor. CAB51127 represents a human QA79 membrane protein. AAD50978 represents a human sialic acid binding Ig-like lectin-5.

Fig. 3 shows the structure of HRC12337. In this figure, a sialic acid binding motif shows that an arginine residue essential for the binding to a sialic acid-containing sugar chain is conserved.

Fig. 4 shows the expression profile of the hrc12337 gene in human organs and various cells.

Fig. 5 shows a standard curve of a sandwich ELISA using an anti-HRC12337 antibody.

Fig. 6 shows the results of the determination of HRC12337 concentration in culture supernatants of various cell strains in a sandwich ELISA using an anti-HRC12337 antibody.

Fig. 7 shows the results of the determination of HRC12337 concentration in sera of various patients in a sandwich ELISA system using an anti-HRC12337 antibody.

Fig. 8 shows analytical results of a COS cell transformant by flow cytometry using an OG-labeled anti-HRC12337 antibody. Awhite background region shows the results of the staining with an OG-labeled F1114-1-2 antibody and a black background region shows the results of the staining with an OG-labeled control antibody.

Fig. 9 results analytical results of the expression of HRC12337 on human peripheral blood monocytes by a flow cytometry. A black background region shows the results of non-stimulated peripheral bloodmonocytes stained with an OG-labeled F1114-1-2 antibody. A white background solid line region illustrates the results of PMA+Ionomycin stimulated peripheral bloodmonocytes stained with an OG-labeled F1114-1-2 antibody. A white background dotted line region shows the results of PHA-L stimulated peripheral blood monocytes stained with an OG-labeled F1114-1-2 antibody.

Fig. 10 shows the action of HRC12337-Fc on a mixed lymphocyte reaction. An amount of bromodeoxyuridine incorporated in cellular DNA upon mixed lymphocyte reaction was determined as an index.

Fig. 11 shows the action of HRC12337-Fc on a mixed lymphocyte reaction. An IL-2 concentration in a culture serum upon a mixed lymphocyte reaction was determined as an index.

EXAMPLES

The present invention will be described in more detail by referring to the following examples which are not to be construed as limiting the scope of the invention.

Example 1 : Cloning of gene hrc12337

(1) Construction of full length cDNA library according to oligocapping method

A poly(A) $^+$RNA was prepared from a human renal cortical epithelial cell according to the method of Sambrook et al. (Molecular Cloning. A LaboratoryManual, 2nd edition, Cold Spring Harbor Laboratory, Cold Spring Harbor, NY) using an oligo (dT)-cellulose. Next, 5 to 10 µg of the poly (A) $^+$RNA was reacted with 1.2U of Bacterial Alkaline Phosphatase (hereinafter abbreviated as "BAP") (TaKaRa) in a buffer containing 100mM of Tris-HCl (pH 8.0), 5mM of 2-mercaptoethanol and 100U of RNasin (Promega) at 37°C for 40 minutes so as to dephosphorylate the poly (A) $^+$RNA having no cap structure. Thereafter, the reaction liquid was extracted with a mixture of phenol and chloroform (1:1) twice and the poly(A) $^+$RNA was collected as ethanol precipitates. The thus-collected poly (A) $^+$RNA was treated with 20U of Tobacco acid pyrophosphatase (hereinafter abbreviated as "TAP") (Maruyama and Sugano, Gene, Vol. 138, pp. 171-174 (1994)) in a buffer containing 50mM of sodium acetate (pH 5.5), 1mM of EDTA, 5mM of 2-mercaptoethanol and 100U of RNasin at 37°C for 45 minutes so as to remove the cap structure. Thereafter, the reaction liquid was extracted with a mixture of phenol and chloroform (1:1) twice and subjected to ethanol precipitation to collect a BAP-TAP treated poly(A) $^+$RNA.

2 to 4 µg of the thus-collected poly(A) $^+$RNA was ligated to 0.4 µg of 5'-oligomer (5'-AGC AUC GAG UCG GCC UUG UUG GCC UACUGG-3') . This reaction was conducted with 250U of RNA ligase (TaKaRa) in a buffer containing 50mM of Tris-HCl (pH 7.5), 5mM of $MgCl_2$, 5mM of 2-mercaptoethanol, 0.5mM of ATP, 25% of PEG8000 and 100U of RNAsin at 20°C for 3 to 16 hours. Thereafter, the oligomer unreacted was removed, and a cDNA was synthesized. That is, 2 to 4 µg of the oligocapped poly (A) $^+$RNA was mixed with 10pmol of a dT adaptor-primer (5'-GCG GCT GAA GAC GGC CTA TGT GGC CTT TTT TTT TTT TTT TTT-3') and the mixture was reacted with SuperScript II RNase $H^-$ Reverse Transcriptase (Gibco BRL) in a buffer attached thereto at 42°C for 1 hour.

The reaction was conducted with 15mM of NaOH at 65°C for one hour to remove the RNA as a template and then a cDNA was amplified. 1 µg of the cDNA synthesized from the oligocapped poly(A) $^+$RNA was mixed with 16 pmol of a sense primer 1 (5'-AGC ATC GAG TCG GCC TTG TTG-3') and an antisense primer 1 (5'-GCG GCT GAA GAC GGC CTA TGT-3') and amplified using XL PCR kit (Perkin-Elmer) . The reaction condition for PCR was 5 to 10 cycles, each cycle comprising heating at 94°C for 1 minute, at 58°C for 1 minute and at 72°C for 10 minutes. The PCR product was extracted with a mixture of phenol and chloroform (1:1) and collected as ethanol precipitates. Thereafter, the thus-collected product was digested with SfiI and subjected to electrophoresis on agarose gel to separate a cDNA of 1,000 bp or higher. This cDNA was inserted into DraIII site of pME18S-FL3 (GenBank accession No. AB009864) which was an expression vector for mammalian cells . Since this DraIII site of pME18S-FL3 was asymmetrical, the terminal of the cDNA fragment was a SfiI site complementary thereto and therefore the cDNA fragment was inserted unidirectionally.

(2) Sequencing of cDNA clone and analysis of information about its deduced protein

A plasmid was prepared from the cDNA library constructed by the method described in the above section (1) by means of a pI-100 robot (KURABO). Each clone was sequenced and the resultant sequence was used as data base. A sequence reaction was conducted using AutoCycle sequencing kit (Amersham Pharmacia) and R.O.B. DNA processor (Amersham Pharmacia) according to the provider's protocol, and a base sequence determined using with in ALF DNA sequencer (Amersham Pharmacia)

The clone C-HRC12237 obtained by the method (1) contained a cDNA comprising the nucleotide sequence of 1523 base pairs in full length represented by SEQ ID NO:3, containing an open reading frame (ORF) comprising the nucleotide sequence of 984 base pairs represented by SEQ ID NO:1, encoding a novel protein comprising 328 amino acids represented by SEQ ID NO:2. This plasmid C-HRC1233 was deposited in International Patent Organism Depositary (IPDO) (Tsukuba City, Ibaragi Prefecture, Japan) of National Institute of Advanced Industrial Science and Technology as FERM BP-7882 on February 14, 2001.

(3) Homology analysis of cDNA clone and its deduced protein

For searching the homology, BLAST (J. Mol. Evol., Vol. 36, pp. 290-300 (1993) ; J. Mol. Biol., Vol. 215, pp. 403-10 (1990)) was conducted to search a local agreement in sequence.

The protein sequence deduced from the C-HRC12337 full length cDNA sequence was subjected to BLAST homology searching with Genbank protein data base (http://www.ncbi.nlm.nih.gov/) using a blastp program searching a homology. The homology with human CD33 antigen (GenBank accession No._Np 001763) was found across 199 residues at a level of 43%. The homology with human D-siglec precursor (GenBank accession No. Np_057627) was found across 260 residues at a level of 40%. The homology with human QA79 membrane protein (GenBank accession No. CAB51127) was found across 260 residues at a level of 40%. The homology with human sialic acid binding Ig-like lectin-5 (GenBank accession No. ATT50978) was found across 201 residues at a level of 43%.

Further, the deduced protein sequence and a protein sequence obtained by the homology search (GenBank accession No. NP_00763, NP_057627, CAB51127, AAD50978) were subjected to multiple alignment using a multiple alignment program ClustalW (Nucleic Acids Res., Vol. 22, pp. 4673-80 (1994)). The results are shown in Figure 2.

Motifs are represented as consensus sequences of functional sites (for example, an active site such as an enzyme; a binding site such as a ligand or an effector; a modifying site such as a phosphorylation) identified via experiment and the like. Since especially important functional sites are often conserved even after evolution, it is used as an index characterizing a conserved sequence specifically appearing in a protein expressing certain function and also an analogous protein family. Thus, it is expected to lead an interpretation which directly relates to functions by searching a motif rather than a homology.

In PROSITE data base (http://www.expasv.ch/prosite/) which is a motif data base, a motif is represented by a pattern of a consensus sequence. Alternatively, it is represented by a profile in which a score based on an appearance frequency or the like is expressed with a matrix for each amino acid to each position within the motif.

As to the functions of the deduced protein sequence, patterns were searched for PROSITE data base. As the result, motifs where asparagine at the positions of Nos. 172 and 312 are sites to binding sugar chain.

Pfam (http://www.sanger.ac.uk/Pfam/) which is a protein domain data base was searched according to HMMER (R.Durbin, S.Eddy, A.Krogh, G.Mitchison, Cambridge University Press (1998)) which is a program searching for a homology using a hidden Markov model. As the result, an immunoglobulin domain [Pfam Accession No. PF00047] was found at amino acid Nos. 57 to 144 and Nos. 187 to 239 as a significantly homologous domain.

The assessment of a transmembrane helix was conducted according to tmap (J. Mol. Biol. , Vol. 237, pp. 182-92 (1994) which is a transmembrane assessment program using a weighted matrix. As the result, a transmembrane region was assessed in 29 amino acid residues of amino acid Nos. 255 to 283. And, according to SOSUI program, a transmembrane region was assessed in amino acid residues Nos. 255 to 283.

The assessment of a signal peptide was conducted according to sigcleave (NucleicAcidResearch, Vo. 14, pp. 4683-4690 (1986) which is a program assessing signal peptide cleaving sites using a weighted matrix. As the result, a signal peptide was assessed in 14 amino acid residues of amino acid Nos. 7 to 20. And, according to SOSUI program, a signal peptide was assessed in an amino acid sequence of amino acid Nos. 3 to 22.

Example 2 : Confirmation of expression profile by PCR

Real time PCR (Journal of Clinical Microbiology, Vol. 38, pp. 586-590 (2000) was conducted by contracting to the outer research institute according to the following procedures.

A total RNA was prepared from human coronary artery endothelial cells (HCAEC), human leukocytes and human placental tissue according to a routine method. While, a total RNA was prepared from leukocytes stimulated with 5 μg/ml of PHA for 24 hours according to a routine method. And, a total RNA of each of lung, kidney, pancreas, liver, colon, small intestine, thymus, spleen, heart, uterus, testis, prostate, skeletal muscle and brain of human was purchased from Clontech. A single strand cDNA was synthesized from 1 to 2 μg of each total RNA according to a routine method using an oligo(dT)- primer. As a reverse transcriptase, SuperScript II RNase H-Reverse Transcriptase (Invitrogen) was used. Real time PCR was conducted using the thus-synthesized cDNA as a template to confirm the expression of hrc12337 into mRNA. Real time PCR was conducted using Light Cycler (Rosche Diagnostics) according to the provider's protocol. As primers for elongation, a sense primer (5'-CAC CAA CAT CCA TTT CAG C-3') and an antisense primer (5'-ATG GTT GTC CAC ATA GGA GG-3') were used. And as hybridization probes, an oligomer comprising 20 mer (5'-TGT GCC AGC CAA GCC TCG-3') labeled with Red 640 at 5'-terminal and an oligomer comprising 20 mer (5'-TGC CCG AGA ACC AGG ACT GG-3') labeled with FITC at 3'-terminal were used. As the result, the expression of hrc12337 was confirmed in spleen, PHA-stimulated leukocytes, uterus and testis. Especially, significantly higher expression was found in PHA-stimulated leukocytes (Fig. 4).

Example 3 : Expression of HRC12337 protein

(1) Expression of HRC12337 protein of soluble type

A chimeric protein having a His tag linked to C-terminus of its extracellular domain was expressed as the HRC12337 protein of soluble type. An expression plasmid was constructed according to the following method. First, a senseprimer (5'-CGT TAC AGA TCC AAG CTC TG-3') and an antisense primer (5'-CCG CTC GAG CCA GGT AGA CGC TGG CCT-C') were designed, and the PCR reaction was conducted using Pyrobest DNA Polymerase (TaKaRa) and C-HRC12337. The PCR reaction comprised 25 cycles, each cycle comprising heating at 98°C for 10 seconds, at 55°C for 30 seconds and at 72°C for 90 seconds. The resultant PCR product of about 1.1 kb was digested with XhoI and subjected to electrophoresis on agarose gel to collect a DNA fragment of about 0.9 kb. While, a pcDNA3.1(+)Myc-His C was digested with XhoI and then dephosphorylated. This vector and the above PCR product were ligated. A competent cell JM109 was transformed according to a routine method to construct a HRC12337-His tag protein expression plasmid.

12.5 µg of the resultant plasmid and 50 µl of FuGNEN6 (Rosche Diagnostics) were mixed according to the provider's protocol, which was added to COS-1 cells grown in a semiconfluent condition in a 150cm$^2$ flask. After the cells were cultured at 37°C in 5% $CO_2$ for 72 hours, a supernatant was harvested. Thereafter, the HRC1237 protein-His tag protein (hereinafter sometime referred to "HRC12337-His") was purified using His-Trap Kit (Amersham Pharmacia Biotech) according to Guide for Carrying Bio Basic Experiments 2000-2001 (Amersham Pharmacia Biotech). An amino acid sequence of the HRC12337-His is shown in SEQ ID NO:6.

(2) Expression of full length HRC12337 protein

A C-HRC12337 plasmid was introduced into COS cells according to the following method so as to express a full length protein.

50 µl of FuGENE6 (Rosche Diagnostics) and 12.5 µg of the above plasmid DNA were mixed according to the provider's protocol, which was added to COS cells grown in a semiconfluent condition in a 150cm$^2$ flask. After the cells were cultured at 37°C and 5% $CO_2$ for 72 hours, cells expressing HRC12337 protein were collected and subjected to an activity assay as described in Example 5.

(3) Preparation of HRC12337-Fc

A chimeric (fused) protein comprising an extracellular domain of the HRC12337 protein and a human IgG Fc fragment was prepared. An expression plasmid of HRC12337-Fc which was a chimeric protein comprising an extracellular domain of the HRC12337 protein and a human IgG Fc fragment was constructed according to the following method. A sense primer (S'-CGT TAC AGA TCC AAG CTC TG-3') and an antisense primer (5'-CGC GGA TCC CAG GTA GAC GCT GGC CT-3') were synthesized, and the PCR reaction was conducted using Pyrobest DNA Polymerase (TAKARA) and C-HRC12337 as a template. The PCR reaction comprised 25 cycles, each cycle comprising heating at 98°C for 10 seconds, at 55°C for 30 seconds and at 72°C for 1 minute. The resultant PCR product of about 1.1 kb was digested with EcoRI and BamHI and subjected to electrophoresis on agarose gel to collect a DNA fragment of about 0.9 kb. A pM1304 described in WO 97/42319 was digested with EcoRI and BamHI and ligated to the above DNA fragment. A competent cell HB101 (TAKARA) was transformed according to a routine method to construct a HRC12337-Fc expression plasmid. The resultant expression plasmid was introduced into COS cells according to the following method. That is, 50 µl of FuGENE6 (Rosche Diagnostics) and 12.5 µg of each of the plasmid DNA described above were mixed according to the provider's protocol, which was added to COS cells grown in a semiconfluent condition in a 150 cm$^2$ flask. After the cells were cultured at 37°C in 5% $CO_2$ for 3 days, a supernatant was harvested. The HRC12337-Fc contained in the culture supernatant was purified through a Protein A column. The thus-purified HRC12337-Fc was subjected to SDS-PAGEelectrophoresisandthendetectedbywesternblotting using an anti-Fc antibody (anti-human IgA, IgG, IgM, Kappa, Lambda antibody labeled with peroxidase; DAKO). An amino acid sequence of HRC12337-Fc is shown in SEQ ID NO:7.

Example 4 : Preparation of anti-HRC12337 antibody

(1) Preparation of partial peptide of HRC12337

In order to prepare an antibody against HRC12337, a peptide sequence which was expected to be exposed to a surface of a protein using a program of anticipating a secondary structure of Chou-Fasman and Robson. As the result, it was judged that among N-terminated amino acids, a sequence of amino acid Nos. 24 to 39 (KIDTTENLLNTEVHSS) is an α-helix containing structure having higher hydrophilicity so that it can be used for immunization. Since it is expected that a sequence of amino acid Nos. 64 to 75 (CTFTHPHRHYDG) and a sequence of amino acid Nos. 121 to 142 (RRNDLSLRVERLALADDRRYFC) take a combination structure partially having a turn structure, it is estimated that they are exposed to a surface of a protein so that it can be used for immunization. In order to bind to a carrier protein, cysteine was bound to the C-terminus of the KIDTTENLLNTEVHSS peptide. Apeptidewas synthesized in ABI 432A peptide synthesizer (Applied Biosystems) and the resultant peptide was cleaved from a resin according to a routine method, deblocked and purified through a C18 reverse phase HPLC (CAPCELL-PAK; Shiseido Co.).

(2) Preparation of peptide antigen of HRC12337 with carrier

The thus-synthesized peptide was dissolved in a distilled water at a rate of 10 mg/ml and then mixed with an equal amount of 10mg/ml of maleimidated keyhole limpet hemocyanine (PIERCE) . After the reaction was conducted at room temperature for 2 hours, it was passed through a NAP-10 column (Pharmacia) to desalt. A protein concentration used was an used amount of KLH divided by a volume of a liquid.

(3) Preparation of an antibody against peptide

20 μg of each of the thus-prepared peptide antigens with carrier was dissolved in 100 μl of a saline solution and mixed with an equal amount of Freund's complete adjuvant (DIFCO) . The mixture was intraperitoneally administered to a BALB/c female mouse of 5 week-old, After two weeks, 20 μg of the peptide antigen with carrier was dissolved in 100 μl of saline solution, mixed with an equal amount of Freund's complete adjuvant (DIFCO) and the mixture was intraperitoneally administered in the same way. While, 100 μg of each of the thus-prepared peptide antigens with carrier was dissolved in 100 μl of saline solution and mixed with an equal amount of Freund's complete adjuvant (DIFCO). 100 μl of the mixture was injected to each hind foot pad of a Wistar female rat of 8 week-old. After one week from the second administration (in case of the mouse) or two weeks from the primary administration (in case of the rat), a blood was sampled from fundus oculi and tested for the increase in antibody titer by a western blooting. That is, the purified HRC12337-His was subjected to electrophoresis on 4 to 20% SDS-polyacrylamide gel (TEFCO) and transfered on a PVDF membrane according to the method of Millipore. After the transfer, the PVDF membrane was blocked with a 0.076M phosphate buffer (pH 6.4) (hereinafter abbreviated as "PBS") containing 5% skim milk and 0.05% Tween 20. After the sampled antiserum was diluted 500-fold with a 0.076M phosphate buffer (pH 6.4) containing 0.5% BSA and 0.05% Tween 20 , it was reacted with the above-transfered PVDF membrane at 4°C overnight. The membrane was washed three times with PBS (pH6.4) containing 0.05% Tween 20. While, a peroxidase-labeled anti-mouse immunoglobulins antibody (DAKO) or a peroxidase-labeled anti-rat immunoglobulins antibody (DAKO) was diluted with PBS (pH 6.4) containing 0.5% BSA and 0.05% Tween 20 500-fold. The thus-diluted antibody was reacted with the membrane at room temperature for one hour. The membrane was washed similarly three times and detected using a chemiluminescent reagent ECL (Amersham). As the result, a band was detected near about 37 kDa and the increase in antibody titer was confirmed. Among rats showing the increase in antibody titer, a rat to which a sequence of amino acid Nos. 24 to 39 (KIDTTENLLNTEVHSS) was administered showed a especially high titer. From this rat, an iliac lymph node was extracted to be used for cell fusion. That is, lymphocytes were separated from the lymph node using a cell strainer (Falcon), mixed with myeloma cells (Sp2/O-Ag14) and then they were subjected to cell fusion using polyethylene glycol according to the method described in "Introduction of Experimental Procedures of Monoclonal antibodies", written by Tamie ANDOH and Joh CHIBA, p. 83, published by Kodansha Ltd. (1991). Hybridomas was selected using a HAT medium. After one week, a hybridoma producing the desired antibody was screened. While, the antigen was finally administered to the mouse having the increased antibody titer. After three days, lymphocytes were separated from the spleen cell, mixed with Sp2/O-Ag14 and then they were subjected to cell fusion using polyethylene glycol according to the method described in "Introduction of Experimental Procedures of Monoclonal antibodies", written by Tamie ANDOH and Joh CHIBA, p. 83, published by Kodansha Ltd. (1991). Hybridomas was selected using a HAT medium. After one week, a hybridoma producing the desired antibody was screened. Finally, 100 μg of the antigen was intraperitoneally administered to the mouse. After three days, lymphocytes were separated from the spleen, mixed with P3x63-Ag. 8.U.1 in a ratio of 10:1 and then they were subjected to cell fusion using polyethylene glycol. Hybridomas were selected and after one week, a hybridoma producing the desired antibody was screened. As the result, a well reacting with HRC12337 was cloned by a limiting dilution method.

For screening, the purified HRC12337-His was diluted with a 0.01M carbonate buffer (pH 9.5) to 1 μg/ml and 50 μl/well of the thus-diluted HRC12337-His was added to each well in an immunoplate (Maxisorb; NUNC). After the reaction was conducted at 37°C for one hour, the wells were washed with an ion-exchanged water five times. Thereafter 100 μl of PBS (pH 6.4) containing 0.5% BSA was added to each well so as to be blocked. Next, the culture supernatant was added to each well, reacted at 37°C for one hour and then washed with saline solution containing 0.05% Tween 20 three times. While, a peroxidase-labeled anti-mouse immunoglobulins antibody (DAKO) or a peroxidase-labeled anti-rat immunoglobulins antibody (DAKO) was diluted 1000-fold with PBS (pH 6.4) containing 10% rabbit serum . 50 µl of this dilution was added to each well . After the reaction was conducted at 37°C for one hour, the well was washed similarly five times and a tetramethylbenzidine solution containing 0.01% hydrogen peroxide was added to each well. The reaction was conducted at room temperature for 10 minutes and then stopped by adding a 0.5M sulfuric acid solution. An absorbance at 450 nm was determined by a plate spectrophotomerty (NJ-2100; Nippon Intermed). As the result, a cell reacting with HRC12337-His was selected and cloned by a limiting dilution method. After 10 days, the screening was conducted similarly. From the mice, hybridoma 5 clones (F1105-1 to 5) which produced monoclonal antibodies reacting with HRC12337-His were obtained. From the rats, hybridoma 2 clones (F1114-1-2 and F1114-4-1) whichproduced monoclonal antibodies reacting with HRC12337-His were obtained. Each of the thus-selected hybridomas were cultured in a 10% FCS/RPMI-1640 medium (GIBCO) and then in a Hybridoma-SFM medium (GIBCO) to produce antibodies. The antibodies were purified using Prosep-A or Prosep-G column (Millipore). Among the resultant antibodies, a subtype of the F1114-1-2 antibody showing especially high reactivity was determined using a rat typing kit (ZYMED). As the result, the subtype was IgG 2b.K.

(4) Preparation of polyclonal antibody

In order to prepare a rabid polyclonal antibody against HRC12337 protein, peptide antigens with carrier(each 40 µg) and an equal amount of Freund's adjuvant were mixed and 1 ml of the mixture was intradermally applied to a back of a female New Zealand white rabbit (Kitayama Labes Co., Ltd.; 2.0 to 2.49 kg). After two weeks, an antigen and an equal amount of Freund's incomplete adjuvant (DIFCO) were mixed and the mixture was applied similarly. After additional one week, a blood was taken via auricular vein to prepare an antiserum. 30 µg/body of HRC12337 purified similarly was applied several times to prepare an antiserum. The resultant antiserum was tested for the increase in antibody titer using a plate on which HRC12337-His was immobilized as described in (3). As the result, a slight increase in antibody titer was detected, but the antibody titer was not so high that it can be used in immunohistological staining, immunoassay and the like. Since it is thought that causes for which the increase in antibody titer is inhibited are that HEC12337 has high homology and low antigenicity in mammalian animals, the use of a chicken which is a species very remote from mammalian animals such as human, rabbit and the like was attempted in the preparation of an antibody. As an antigen to be administered, a fused protein HRC12337-Fc in which Fc of human gamma-globulin having high antigenicity as described in Example 3 was linked was used. Immunization of chicken contracted to Sawaday Technology. After 60 days after the administration of the antigen, all blood was taken from the chicken to obtain an antiserum. An antibody titer was determined in the same way. But, an antibody having a high titer such that it can be used in immunohistological staining, immunoassay and the like could not be obtained.

(5) Preparation of anti-HRC12337 monoclonal antibody

20 µg of the purified HRC12337 protein (HRC123337-His or HRC12337-Fc) mixed with an equal amount of Freund's complete adjuvant (DIFCO) was intraperitoneally administered to a female BALB-c mouse of 6-week old. After two weeks from the initial administration, 20 µg of the antigen dissolved in saline solution was mixed with an equal amount of Freund's incomplete adjuvant (DIFCO) and then the mixture was intraperitoneally administered to the mouse. After one week, the increase in antibody titer was tested according to the method described in Example 2 and finally 100 µg of the antigen was intraperitoneally administered to the mouse. After three days, a spleen was extracted. Lymphocytes were separated from spleen, mixed with P3x63-Ag.8.U.1 in a ratio of 10:1 and then subjected to cell fusion using polyethylene glycol. Hybridomas were selected using a HAT medium, among which a hybridoma producing the desired antibody was screened after one week. Wells producing anti-HRC12337 antibodies were re-screened according to the method described in (3) and a well which reacted with HRC12337 was cloned by a limiting dilution method. The resultants clones were screened to obtain ten anti-HRC12337 monoclonal antibodies (F1144-1-1 to 10). The hybridomas were cultured in a 10% FCS/RPMI1640 medium (GIBCO) and then they were cultured in a Hybridoma-SFM medium (GIBCO) so as to produce antibodies. The antibodies were purified through a Prosep-A column (Millipore) . The resultant antibody was tested for antibody titer, but its titer was not so high that it can be used in immunohistological staining, immunoassay and the like.

A rat monoclonal antibody was prepared according to the following method. First, 100 µg of the HRC12337-Fc protein was dissolved in 100 µl of saline solution and mixed with an equal amount of Freund's complete adjuvant (DIFCO). 100 µl of the mixture was applied to each hind foot pad of a Wistar female rat of 8 week-old. After 2 weeks, an iliac lymph node was extracted to be used for cell fusion. That is, lymphocytes were separated from the lymph node using a cell strainer (Falcon), mixed with myeloma cells (Sp2/O-Ag14) and then they were subjected to cell fusion using polyethylene glycol according to the method described in "Introduction of Experimental Procedures of Monoclonal antibodies", written by Tamie ANDOH and Joh CHIBA, p. 83, published by Kodansha Ltd. (1991). Hybridomas was selected using a HAT medium. After one week, a hybridoma producing the desired antibody was screened.

Screening was conducted according to an ELISA directly immobilizing HRC12337-His protein on a plate as described in the above (3). As the result, wells containing hybridomas which produced antibodies reacting with HRC12337 protein were selected and then the hybridomas were cloned by a limiting dilution method. After 10 days, a hybridoma which produced an antibody binding to the HRC12337 protein was similarly screened. As the result, a rat monoclonal antibody, F1112-3-1 antibody, binding to the HRC12337 protein was obtained. This hybridoma was cultured in a 10% FCS/RPMI-1640 medium (GIBCO) and then a Hybridoma-SFM medium (GIBCO) to produce an antibody. The antibody was purified using a Prosep-G column (Biopressesing).

Example 5 : Confirmation of activity of HRC12337 in binding to sialic acid-containing sugar chain

The recombinant COS cells expressing HRC12337 prepared in Example 3 were incubated with human erythrocytes in DMEM + 0.2% BSA at 37°C for one hour and then unbound erythrocytes were removed by washing. The remaining cells were immobilized with 0.25% of glutaraldehyde to observe the binding of COS cells with erhythrocytes. As a control group, recombinant COS cells not expressing HRC12337 were provided and treated in the same way as described in above. As the result, a significant formation of rosettes was observed in the group of the recombinant cells expressing HRC12337 as compared with the control group.

Example 6 : Preparation of sandwich ELISA

A sandwich ELISA was prepared using a F1113 antibody solid phase/a F1114-1-2 antibody labeled with peroxidase as follows. First, 1 mg of peroxidase (Toyobo Co., Ltd.) was dissolved in a distilled water according to the method of Nakane et al. (J. Histochem. Cytochem., Vol. 22, p. 1084 (1974)), to which 100mM periodic acid dissolved in a distilled water was added and reacted at 25°C for 20 minutes. After the reaction was finished, 1.5% ethyleneglycol was added and reacted at 25°C for 10 minutes. Thereafter, the reaction was dialyzed against an 1mM acetate buffer (pH 4.4). The F1114-1-2 antibody was dialyzed against a 10mM carbonate buffer (pH 9.5). 1 mg of peroxidase activated by adding an 1M carbonate buffer (pH 9.5) was mixed and reacted at 25°C for 2 hours. Further, 4 mg/ml of sodium borohydride was added and reacted at 4°C for 2 hours. The reaction liquid was dialyzed against PBS (pH 6.4) to obtain a peroxidase-labeled F1114-1-2 antibody. A plate on which a F1133 antibody was immobilized was prepared as follows: First a F1133 antibody was diluted with PBS (pH 6.4) to 10 μg/ml and 50 μl of the thus-diluted antibody was added to each well of an immunoplate (Maxisorp; NUNC). It was reacted at 45°C for 30 minutes and then washed with an ion-exchanged water five times. To each well was added 100 μl of PBS (pH 6.4) containing 20% blockace (Snow Brand Milk Products Co., Ltd.), thereby the plate on which F1133 antibody was immobilized was prepared. Standards were prepared by diluting the purified HRC12337-His protein with a rat serum to 8, 16, 31, 63, 125, 250 and 500 ng/ml. As a blank, a rat serum was used. For the determination, the blocking agent of the plate was decated and each 25 μl of the above-prepared standard or the blank and then 25 μl of the peroxidase-labeled F1114-1-2 antibody which was diluted to 5 μg/ml with PBS (pH 7.4) containing 1% BSA were poured and reacted at 4°C overnight. The plate was washed five times with saline solution containing 0.05% of Tween 20 and then a tetramethylbenzidine solution (TMB. BioFX) was added to each well. The reaction was conducted at room temperature for 20 minutes and then stopped by adding a 0.5M sulfuric acid solution. An absorbance at 450 nm was determined in a plate spectrophtometer (NJ-2100; Nippon Intermed) to prepare a standard curve as shown in Fig. 5.

Example 7 : Determination of HRC12337 protein in culture supernatant of different cell strains

A concentration of the HRC12337 protein in a culture supernatant of different cell strains (173 strains) was determined using the assay system as described in Example 6. As the result, the concentration of the HRC12337 protein in a culture supernatant of many cells such as HeLa and A549 cells was the order of 10 ng/ml (in 158 cell strains other than 15 cell strains shown in Fig. 6, the concentration ranged from 0 to 20 ng/ml). Whereas, in cell strains of B-lymphocytes such as Daudi, DG75, Ball cells and the like, the HRC12337 protein in the concentration ranging from about 50 to 100 ng/ml was detected. In megakaryocytic cells, typical example of which is CMK cells, the HRC12337 protein in the concentration of about 50 ng/ml was detected. These results suggests that the HRC12337 protein may contribute to the regulation of B-lymphocytes involved in the production of an antibody and megakaryocytes involved in the production of platelets.

Example 8 : Determination of HRC12337 protein in sera of different patients

40 Healthy subjects (20 males and 20 females) and 103 patients suffering from various diseases (35 diseases) were tested using the assay method system described in Example 6. As the result, the concentration of the HRC12337 protein in the healthy subjects ranged 0 to 50 ng/ml (average 24 ng/ml). No difference between sexes was found. As shown in Fig. 7, there is a tendency that the concentration of the HRC12337 protein in the sera of patients suffering from pollenosis and atopic dermatitis showed higher value. Similarly, there is a tendency that the concentration of the HRC12337 protein in the sera of patients suffering from angiitis showed higher value.

The results obtained by determining the concentration of the HRC12337 protein in culture supernatants of different cell strains and the concentration of the HRC12337 protein in sera of different patients show a possibility that the HRC12337 protein may participate in controlling the differentiation of cells of immune system and blood system. Especially, it became clear that the HRC12337 protein is associated with pollenosis, atopic dermatitis and angiitis. In addition, the above results show that the HRC12337 protein of soluble type is present *in vivo.*

Example 9 : Analysis of cell expressing HRC12337 by flow cytometry

(1) Fluorescent staining of anti-HRC12337 peptide antibody The F1114-1-2 antibody prepared in Example 4 was stained with a fluorescent dye in order to use in a flow cytometry. That is, the F1114-1-2 antibody was labeled with Oregon Green 488 dye using Oregon Green 488 Protein Labeling Kit (Molecular Probe) according to the provider's protocol. Hereinafter the F1114-1-2 labeled with Oregon Green 488 dye is referred to "OG-labeled F1114-1-2". In order to confirm a reactivity of this OG-labeled F1114-1-2 antibody, this antibody was analyzed by a flow cytometry according to the following method. After the C-HRC12337 plasmid was transfected into COS cells according to the method shown in Example 3-(2), the cells were removed in $PBS^-$ containing 0.1% EDTA. The thus-removed COS transformant was incubated with 10 μg/ml of rat IgG on ice for 30 minutes and then washed with $PBS^-$ containing 0.15% of rat serum, 0.15% of mouse serum and 0.1% of EDTA twice. Next, the COS transformant was incubated with the OG-labeled F1114-1-2 in a $PBS^-$ solution containing 3% of rat serum, 3% of mouse serum and 0.1% EDTA on ice for 60 minutes, washed with $PBS^-$ containing 0.15% of rat serum, 0.15% of mouse serum and 0.1% EDTA three times and then analyzed using FACA Calibur (Nippon Becton Dickinson) . As the result, aspecific stainingwiththe OG-labeled F1114-1-2 rather than the OG-labeled control antibody was confirmed and the expression of HRC12337 on a cell membrane was also confirmed (Fig. 8).

(2) Expression of HRC12337 on human peripheral blood monocytes

Monocyte fractions expressing HRC12337 were tested using human peripheral blood monocytes. Human peripheral blood monocytes purchased BIO WHITTAKER (agency: Sanko Junyaku Co., Ltd.) were suspended in a RPMI1640 medium (Sigma) containing 5% of a heat-inactivated FBS and inoculated at $1.0 \times 10^6$ cells/well in a 24-well plate. Optionally, 5 ng/ml of Phorbol 12-myristate 13-acetate (hereinafter abbreviated as "PMA"; Sigma) together with 0.5 μg/ml of Ionomycin (Sigma) or 5 μg/ml of Leucoagglutinin (hereinafter abbreviated as "PHA-L"; Sigma) were added to the medium. After the cells were cultured in 5% $CO_2$ at 37°C for 16 hours, they were analyzed by a flow cytometry in the same way as described in (1) using the OG-labeled F1114-1-2 antibody prepared in (1). As the result, the expression of HRC12337 in peripheral blood monocytes was hardly confirmed when any stimulation was not applied, but it was increased when the stimulation by PMA+Ionomycin or PHA-L was applied (Fig. 9). These results agree with the analysis of the expression profile described in Example 2. Further, peripheral blood monocytes are stained with both a commercially available Phycoerythrin (PE) -labeled cell surface marker and the OG-labeled F1114-1-2. As the result, the expression of HRC12337 was confirmed in a part of CD3 positive cells and its expression level was increased when the stimulation by PMA+Ionomycin or PHA-L was applied. Thus, it is thought that HRC12337 is expressed on activated T-cells. The expression of HRC12337 was not confirmed in CD33 positive cells showing a homology with HRC12337.

Example 10 : Mixed lymphocyte reaction

In order to examine the effect of HRC12337 on lymphocyte functions, HRC12337-Fc was added to a mixed lymphocyte reaction. Human peripheral blood monocytes from different donors which were fractionized by a density gradient centrifugation (CC-2702; Sanko Junyaku Co., Ltd.) were cultured in a RPMI1640 medium (R6504; SIGMA) containing 5% inactivated FBS to a cell concentration of $1 \times 10^6$ cells/ml. HRC12337-Fc was poured in a 96 well plate such that its final concentration became 2 μg/ml, to which 100 μl of the human peripheral blood monocytes from different donors was added. After the cells were cultured in a $CO_2$ incubator for four days, the lymphocyte proliferation was quantified by Cell Proliferation ELISA, BrdU (1647229; Rosche Diagnostic) . An amount of bromodeoxyuridyl (BrdU) incorporated in a cellular DNA was used as an index. As the result, it was shown that HRC12337-Fc promotes the proliferation of human peripheral lymphocytes in a mixed lymphocyte reaction (Fig. 10). Simultaneously, the concentration of various cytokines in the culture supernatant was determined by the ELISAmethod (RPN2752; Amersham Pharmacia). As the result, the increase in the concentration of IL-2 was observed upon the addition of HRC12337-Fc (Fig. 11). Whereas, the change in the concentrations of TNFα and IL-8 by the addition of HRC12337-Fc was not observed.

Example 11 : Production of transgenic mouse

(1) Construction of vector for production of transgenic mice including hrc12337 gene

A plasmid used in the production of transgenic mice was constructed according to the following method. A sense primer (5'-GCT CTA GAA TGG AAA AGT CCA TCT GGC TGC TGG CCT GCT TGG-3') and an antisense primer (5'-CGG GGT ACC TCA CGG TGA GCA CAT GGT GGC TGG TGG GCT CC-3') were synthesized, and the PCR reaction was conducted using Pyrobest DNA Polymerase (TAKARA) and C-HRC12337 as a template. The PCR reaction comprises 25 cycles, each cycle comprising heating at 98°C for 5 seconds, at 55°C for 30 seconds and at 72°C for 90 seconds. The resultant PCR product of about 1.0 kb was digested with XbaI and KpnI and subjected to electrophoresis on agarose gel to collect a DNA fragment. While, an expression vector pM1101 (an expression vector having human Elongation Factor 1α promoter and SV40 poly A addition signal) was digested with XbaI and KpnI and ligated to the above DNA fragment. A competent cell HB101 (TAKARA) was transformed according to a routine method to construct a plasmid for the production of transgenic mice according to a routine method.

(2) Preparation of DNA for injection

In order to remove a sequence from the prokaryotic plasmid, the plasmid vector obtained in the above (1) was digested with restriction enzymes EcoT22I, SalI and BsaI. The resultant DNA fragment of 2.9 kb was used for injection.

(3) Injection

The DNA fragment for injection obtained in the above (2) was injected into male pronucleic oosperm of a C57Black/6 (hereinafter referred to "B6") mouse using a micromanipulator according to a routine method. 453 oosperms in total were injected, among which normally living 428 oosperms were transplanted into oviducts of 17 pseudopregnant (0.5 day) ICR mice. On a calculated date of confinemen, each mouse was subjected to cesarean section and children were breeded by foster parents. As the result, 109 children were obtained from the above-treated germs. 96 children grown to a delactation stage were tested for the transmission of the gene introduced.

(4) Confirmation of subjects as to the transmission of gene

The subjects were tested for the transmission of the gene by a southern blotting as described below.

(i) DNA extraction

On 3rd week from the birth, a tail of each of 96 mice born from the germ to which the gene was injected as described in the above (3) was cut by 5 mm from the tip. The thus-cut tail was treated to extract a DNA according to the following procedure. The tail was treated in 0.5 ml of a histolytic solution (50mM Tris-HCl (pH 8.0), 100mM NaCl, 20mM EDTA, 0.5% SDS, 200 μg/ml Proteinase K) overnight to lyse a tissue. The tissue was treated with phenol and chloroform and then a DNA was precipitated using isopropanol. This DNA was rinsed with 70% ethanol and dissolved in TE.

(ii) Preparation of probe

A site corresponding to a promoter region of Elongation Factor of an expression vector pM1101 was digested with restriction enzymes EcoT221 and XbaI. The resultant DNA fragment was labeled with fluorescein in a gene image (Amersham Pharmacia) according to the provider's protocol to prepare a probe (conveniently hereinafter referred to "EF probe").

(iii) Blotting and detection

The genomic DNA obtained in the above (i) was subjected to electrophoresis, blotting and detection according to the following procedures:

a) treat 5 μg of the genomic DNA with restriction enzyme HindIII (37°C, 3 hours);

b) subject 2.5 μg of the thus-digested DNA to electrophoresis on 0.5% agarose gel in TAE buffer (50V, one hour);
c) treat the agarose gel with an alkaline solution (0.4M NaOH and 0.6M NaCl) (20 minutes x 2);
d) put a nylon membrane (Hybond N; Amersham) on the agarose gel and blot with an alkaline solution (about three hours);
e) rinse the membrane with 2 x SSC;
f) put the membrane in a hybribag (Cosmo Bio) and subject to prehybridization in a hybridization buffer (5xSSC, 0.1% SDS, 5% dextran sulfate, 1/20 volume of a blocking reagent (attached to Gene Image Kit of Amersham), 100 μg/ml of herring sperm DNA (60°C, 20 minutes);
g) subject to hybridization (60°C, O/N after adding 1μl/l of the probe to the hybridization buffer;
h) wash the membrane with 1xSSC, 0.1% SDS for 20 minutes and 0.5xSSC, 0.1% SDS at 60°C for 30 minutes;
i)dilute an anti-fluorescein antibody labeled with alkaline phosphatase (Gene Image Kit) with Buffer A (300mM NaCl, 100mM Tris.HCl (pH9.5)) containing 0.5% BSA 5000-fold and treat at room temperature for one hour;
j) wash the membrane with Buffer A containing 0.3% Tween 20 at room temperature for 10 minutes (three times);
k) rinse the membrane with Buffer A at room temperature;
1) put the membrane in a hybribag and add CDP·Star to expose a film.

In each of the subjects to which the gene was introduced, a band of about 2.9 kb was detected.
From the results of the above analysis, the introduction of the gene was observed in two subjects. The ratio of the number of living subjects to which the gene was introduced to the number of children born was very lower than that of the general case. Thus, it was suggested that an ectopic expression of the hrc12337 gene product during a fetal period may cause a viviparous death with high possibility.

## EFFECT OF THE INVENTION

The protein HRC12337 of the present invention is a protein which may be a cause for the development or the advance of diseases due to abnormal immune functions. It is very useful in the development of medicines for preventing or treating autoimmune disease, immunodeficiency, allergic disease, inflammatory disease such as angiitis, hepatitis, septicus shock and the like, tumor and the like.
And, the gene hrc12337 can be used as an antisense medicine and in gene therapy. The protein HRC12337 is useful as a soluble protein medicine per se or by preparing its soluble fragment (extracellular regions or each domain). Further, an antibody having a reactivity with HRC12337 or its fragment as well as a part of the antibody are useful as an antibody medicine controlling HRC12337 function *in vivo.*

## Claims

1. The following DNA (a) or (b):

   (a) a DNA comprising the nucleotide sequence represented by SEQ ID NO:1, or
   (b) a DNA hybridizable with the DNA of SEQ ID NO:1 under stringent conditions and encoding a cellular adhesion molecule.

2. A protein encoded by the DNA as defined in claim 1.

3. The following protein (a) or (b):

   (a) a protein comprising the amino acid sequence represented by SEQ ID NO:2, or
   (b) a cellular adhesion molecule comprising an amino acid sequence wherein deletion, substitution or addition of one or more amino acids has occurred in the amino acid sequence of SEQ ID NO:2.

4. The following DNA (a) or (b):

   (a) a DNA comprising the nucleotide sequence represented by SEQ ID NO:4, or
   (b) a DNA hybridizable with the DNA of SEQ ID NO:4 under stringent conditions and encoding a protein having at least one activity selected from an activity of binding to a sialic acid-containing sugar chain, an activity of proliferating lymphocytes and an activity of enhancing the production of IL-2.

5. A protein encoded by the DNA as defined in claim 4.

6. The following protein (a) or (b):

   (a) a protein comprising the amino acid sequence represented by SEQ ID NO:5, or
   (b) a protein comprising an amino acid sequence wherein deletion, substitution or addition of one or more amino acids has occurred in the amino acid sequence of SEQ ID NO:5 and having at least one activity selected from an activity of binding to a sialic acid-containing sugar chain, an activity of proliferating lymphocytes and an activity of enhancing the production of IL-2.

7. A fused protein comprising the protein as defined in Claim 6.

8. A recombinant vector including the DNA as defined in claim 1.

9. A transformant produced by transformation with the recombinant vector as defined in claim 8.

10. An antisense nucleic acid inhibiting the expression of the protein as defined in claim 2 or 3.

11. An antisense nucleic acid as defined in claim 10 whose nucleotide sequence is a complementary sequence to the entire or a part of the DNA as defined in claim 1.

12. An antibody against the protein as defined in claim 2 or 3 or its partial peptide.

13. A method of screening an agent capable of controling an activity of the protein as defined in claim 2 or 3, which comprises contacting the protein or the transformant expressing the protein with test substances.

14. A method of screening an agent capable of controling the expression of the DNA as defined in claim 1, which comprises contacting the recombinant vector as defined in claim 8 or the transformant as defined in claim 9 with test substances.

15. A recombinant non-human animal of hrc12337 gene.

16. An assay method for the protein as defined in claim 2 or 3 in a test sample, which comprises using the antibody as defined in claim 12.

**17.** An assay reagent or kit for the protein as define in claim 2 or 3 in a test sample, which comprises containing the antibody as defined in claim 12.

Fig. 1

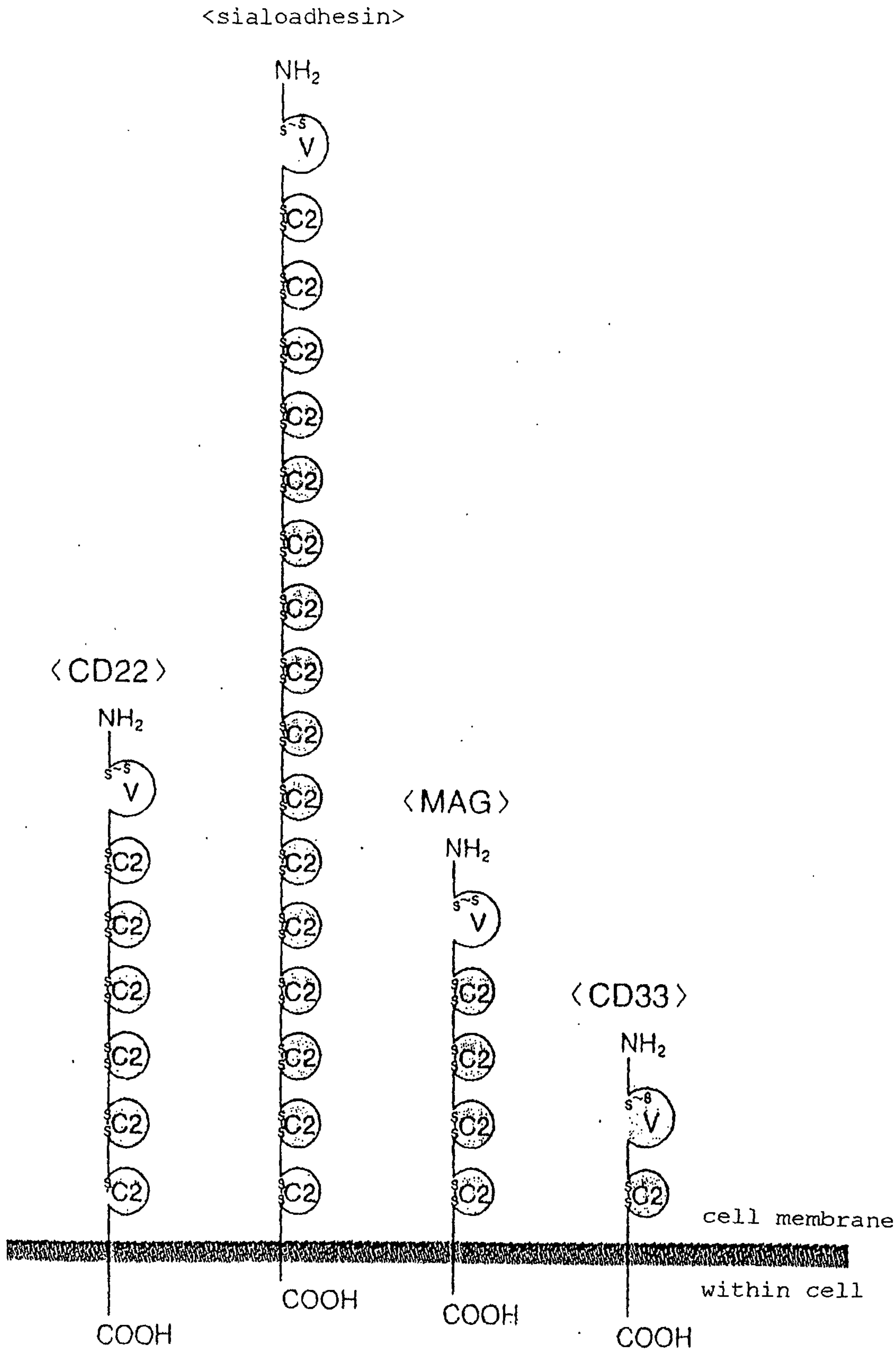
<sialoadhesin>
〈CD22〉
〈MAG〉
〈CD33〉
NH2
V
C2
cell membrane
within cell
COOH

Fig. 2

```
NP_001763      --MPLLLLLPLLWA---------------------GALAMDPNFWLQVQESVTVQEGLCV
AAD50978       --MLPLLLLPLLWG---------------------GSLQEKPVYELQVQKSVTVQEGLCV
NP_057627      --MLLLLLLPLLWGRE----------------RVEGQKSNRKDYSLTMQSSVTVQEGMCA
CAB51127       --MLLLLLLPLLWGRE----------------RVEGQKSNRKDYSLTMQSSVTVQEGMCV
C-HRC12337     MEKSIWLLACLAWVLPTGSFVRTKIDTTENLLNTEVHSSPAQRWSMQVPPEVSAEAGDAA
                     **  * *                              : : :  .*:.: * ..

NP_001763      LVPCTFFHPIPYYDKNSPVHGYWFRE---GAIISGDSPVATNKLDQEVQEETQGRFRLLG
AAD50978       LVPCSFSYPWRSWYSSPPLYVYWFRD---GEIPYYAEVVATNNPDRRVKPETQGRFRLLG
NP_057627      HVRCSFSYPVDSQTDSDPVHGYWFRA---GNDISWKAPVATNNPAWAVQEETRDRFHLLG
CAB51127       HVRCSFSYPVDSQTDSDPVHGYWFRA---GNDISWKAPVATNNPAWAVQEETRDRFHLLG
C-HRC12337     VLPCTFTHPHRHYDG--PLTAIWRAGEPYAGPQVFRCAAARGSELCQTALSLHGRFRLLG
                : *:* :*        *:   *      .        .* ..    .  . :.**:***

NP_001763      DPSRNNCSLSIVDARRRDNGSYFFRMERG-STKYSYKSPQ-LSVHVTDLTHRPKILIPG-
AAD50978       DVQKKNCSLSIGDARMEDTGSYFFRVERGRDVKYSYQQNK-LNLEVTALIEKPDIHFLE-
NP_057627      DPQTKNCTLSIRDARMSDAGRYFFRMEKG-NIKWNYKYDQ-LSVNVTDPPQNLTVTVFQG
CAB51127       DPQTKNCTLSIRDARMSDAGRYFFRMEKG-NIKWNYKYDQ-LSVNVTDPPQNLTVTVFQG
C-HRC12337     NPRRNDLSLRVERLALADDRRYFCRVEFAGDVHDRYESRHGVRLHVTAAPRIVNISVLP-
               :   :: :* :      *   ** *:* . . :  *:  : : :.**   .   : .

NP_001763      ----TLEPGHSKNLTC----SVSWAC--EQGTPPIFSWLSAAPTSLGPRTTHSSVLIITP
AAD50978       ----PLESGRPTRLSC----SLPGSC--EAGPPLTFSWTGNALSPLDPETTRSSELTLTP
NP_057627      EGTASTALGNSSSLSVLEGQSLRLVCAVDSNPPARLSWTWRSLTLYPSQPSNPLVLELQV
CAB51127       EGTASTALGNSSSLSVLEGQSLRLVCAVDSNPPARLSWTWRSLTLYPSQPSNPLVLELQV
C-HRC12337     ----------SPAHAF----RALCTA--EGEPPPALAWSGPALGNSLAAVRSPR--EGHG
                         .   :          .  :  .*  ::*   :     .    .

NP_001763      RPQDHGTNLTCQVKFAGAGVTTERTIQLNVTYVPQ-------------------------
AAD50978       RPEDHGTNLTCQMKRQGAQVTTERTVQLNVSYAPQTITIFRNGIALEILQNTSYLPVLEG
NP_057627      HLGDEG-EFTCRA--QNS--LGSQHVSLNLSLQQE-------------------------
CAB51127       HLGDEG-EFTCRA--QNS--LGSQHVSLNLSLQQE-------------------------
C-HRC12337     HLVTAELPALTHD--------GRYTCTAANSLGRS-------------------------
               :          :                  :   .

NP_001763      -----------NP-------------------TTGIF-----------------------
AAD50978       QALRLLCDAPSNPPAHLSWFQGSPALNATPISNTGILELRRVRSAEKGGFTCRAQHPLGF
NP_057627      ------------------------------------------------------------
CAB51127       ------------------------------------------------------------
C-HRC12337     ------------------------------------------------------------

NP_001763      -P-------------------------------------------------GDGS-----
AAD50978       LQIFLNLSVYSLPQLLGPSCSWEAEGLHCRCSFRAWPAPSLCWRLEEKPLEGNSSQGSFK
NP_057627      -----------------------------------------------------YT-----
CAB51127       -----------------------------------------------------YT-----
C-HRC12337     ------------------------------------------------------------
```

```
NP_001763       ---------------------------------------------GKQETRAGLVHGAIG
AAD50978        VNSSSPGPWANSSLILHGGLNSDLKVSCKAWNIYGSQSGSVLLLQGRSNLGTGVVPAALG
NP_057627       ---------------------------------------------GKMRPVSGVLLGAVG
CAB51127        ---------------------------------------------GKMRPVSGVLLGAVG
C-HRC12337      ----------------------------------------------EASVYLFRFHGASG
                                                              .       . .* *

NP_001763       GAGVTALLALCLCLIFF-IVKTHRRKAARTA-----VGSNDTHPTTGSASPKHQKNSKLH
AAD50978        GAGVMALLCICLCLIFFLIVKARRKQAAGRP-----EKMDDEDPIMGTITSGSRKKPWPD


NP_057627       GAGATALVFLSFCVIFI-VVRSCRKKSARPAADVGDIGMKDANTIRGSALRVT-------
CAB51127        GAGATALVFLSFCVIFI-VVRSCRKKSARPAADVGDIGMKDANTIRGSASQGNLTESWAD
C-HRC12337      ASTVALLLGALGFKALL-LLGVLAARAARRR------PEHLDTPDTPPRSQAQESNYENL
                .: .  *:       :: ::     ::*           .   .   .

NP_001763       GPTETSSCSGAAPTVEMDEELHYASLNFHGMN--PSKD----TSTEYSEVRTQ-
AAD50978        SPGDQASPPGDAPPLEEQKELHYASLSFSEMKSREPKDQEAPSTTEYSEIKTSK
NP_057627       ------------------------------------------------------
CAB51127        D--NPRHHGLAAHSSGEEREIQYAPLSFHKGEPQDLSGQE-ATNNEYSEIKIPK
C-HRC12337      SQMNPRSPPATMCSP---------------------------------------
```

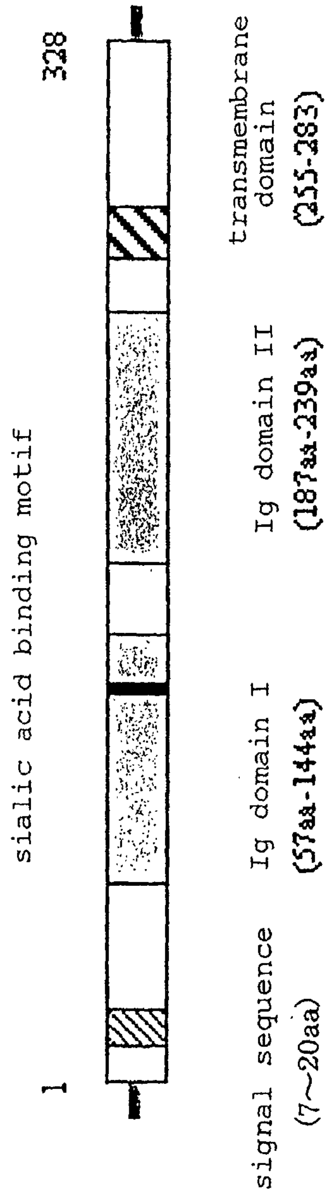
1
328
sialic acid binding motif
signal sequence
(7~20aa)
Ig domain I
(57aa-144aa)
Ig domain II
(187aa-239aa)
transmembrane
domain
(255-283)

Fig. 3

Fig. 4

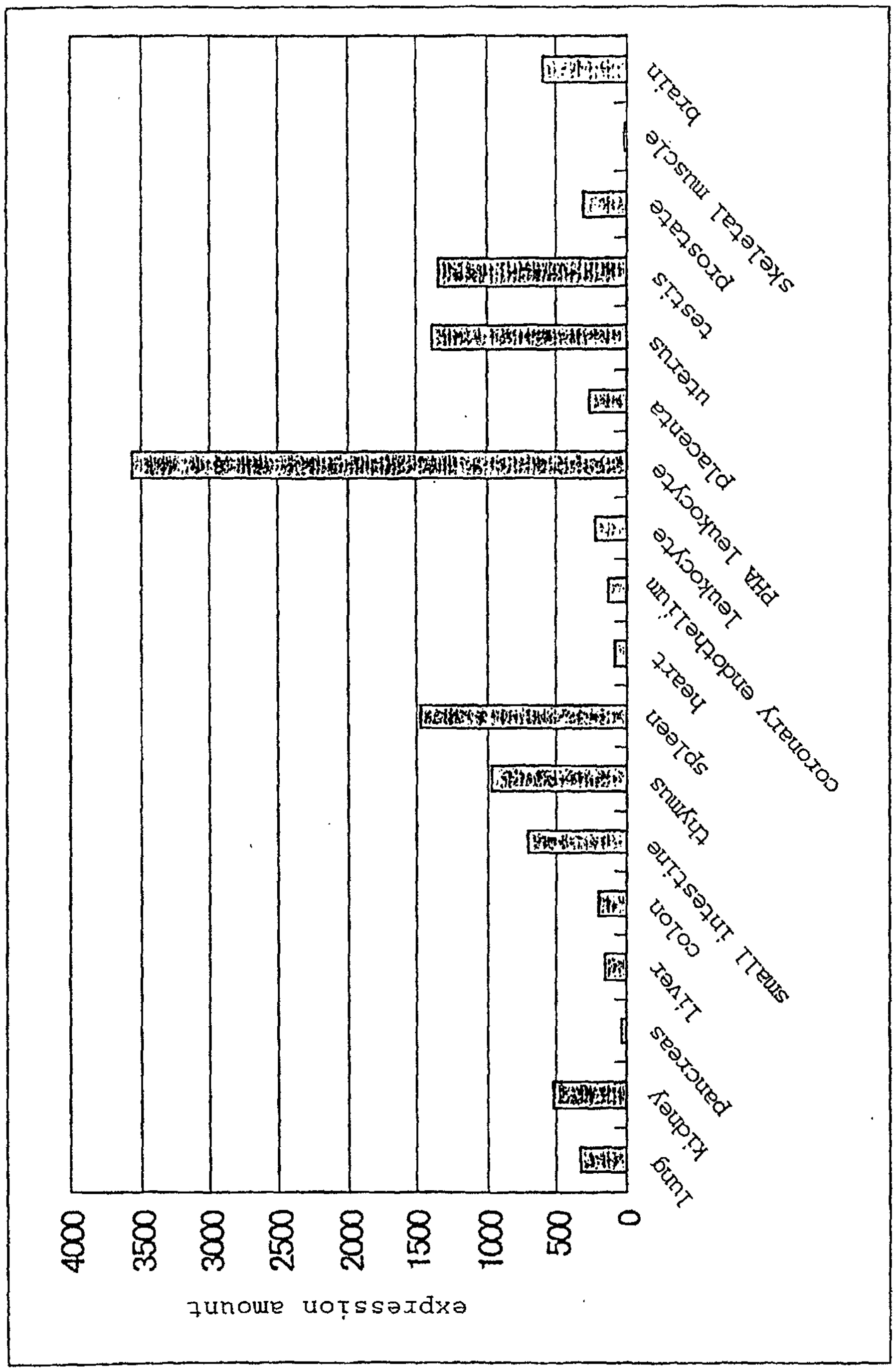
expression amount
0
500
1000
1500
2000
2500
3000
3500
4000
lung
kidney
pancreas
liver
colon
small intestine
thymus
spleen
heart
coronary endothelium
leukocyte
PHA leukocyte
placenta
uterus
testis
prostate
skeletal muscle
brain

Fig. 5

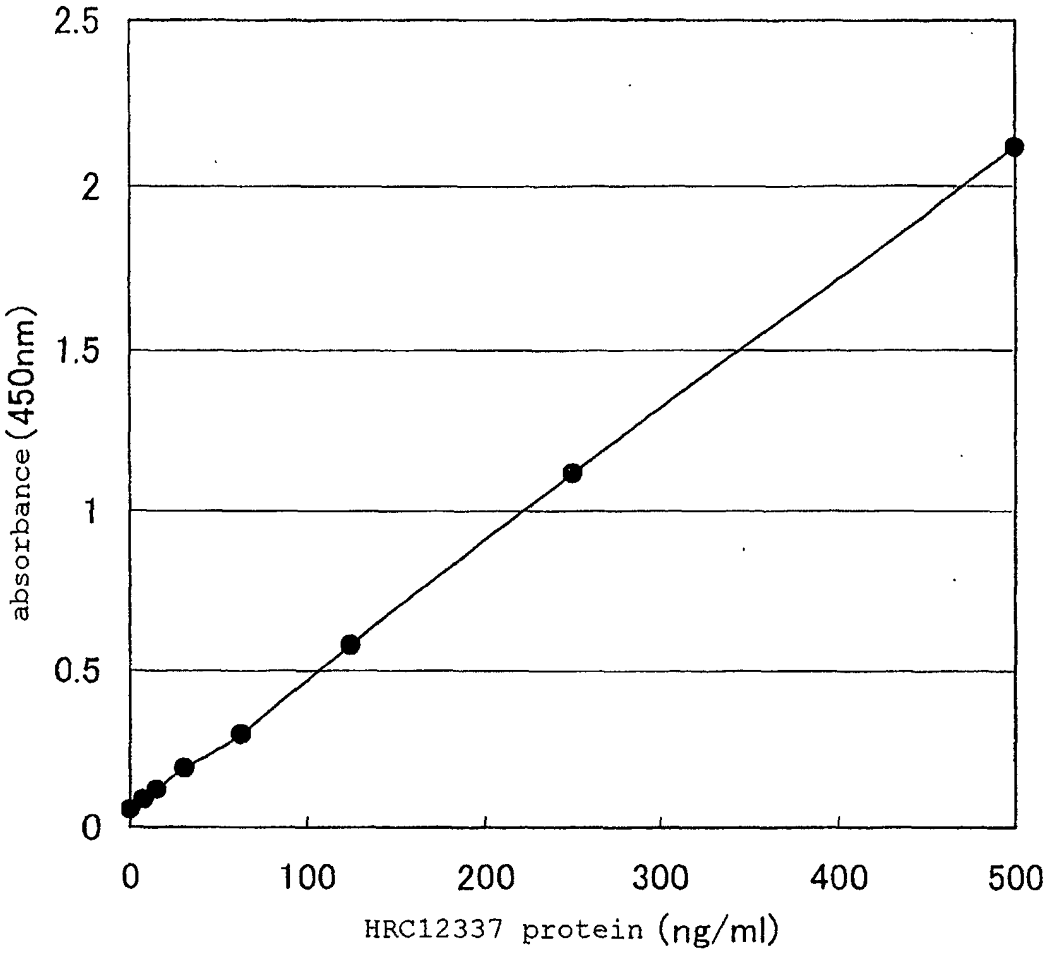
2.5
2
1.5
1
0.5
0
absorbance (450nm)
0
100
200
300
400
500
HRC12337 protein (ng/ml)

Fig. 6

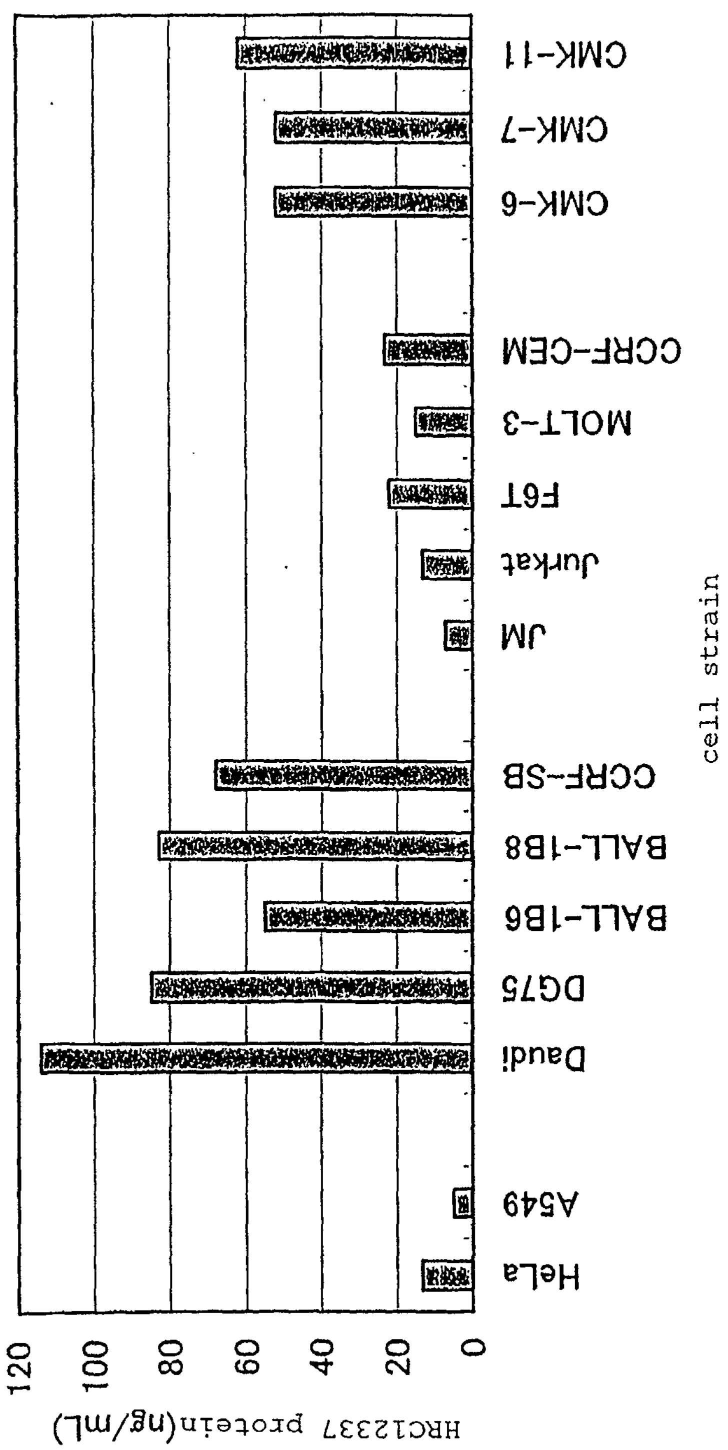
HRC12337 protein(ng/mL)
0
20
40
60
80
100
120
cell strain
HeLa
A549
Daudi
DG75
BALL-1B6
BALL-1B8
CCRF-SB
JM
Jurkat
F6T
MOLT-3
CCRF-CEM
CMK-6
CMK-7
CMK-11

Fig. 7

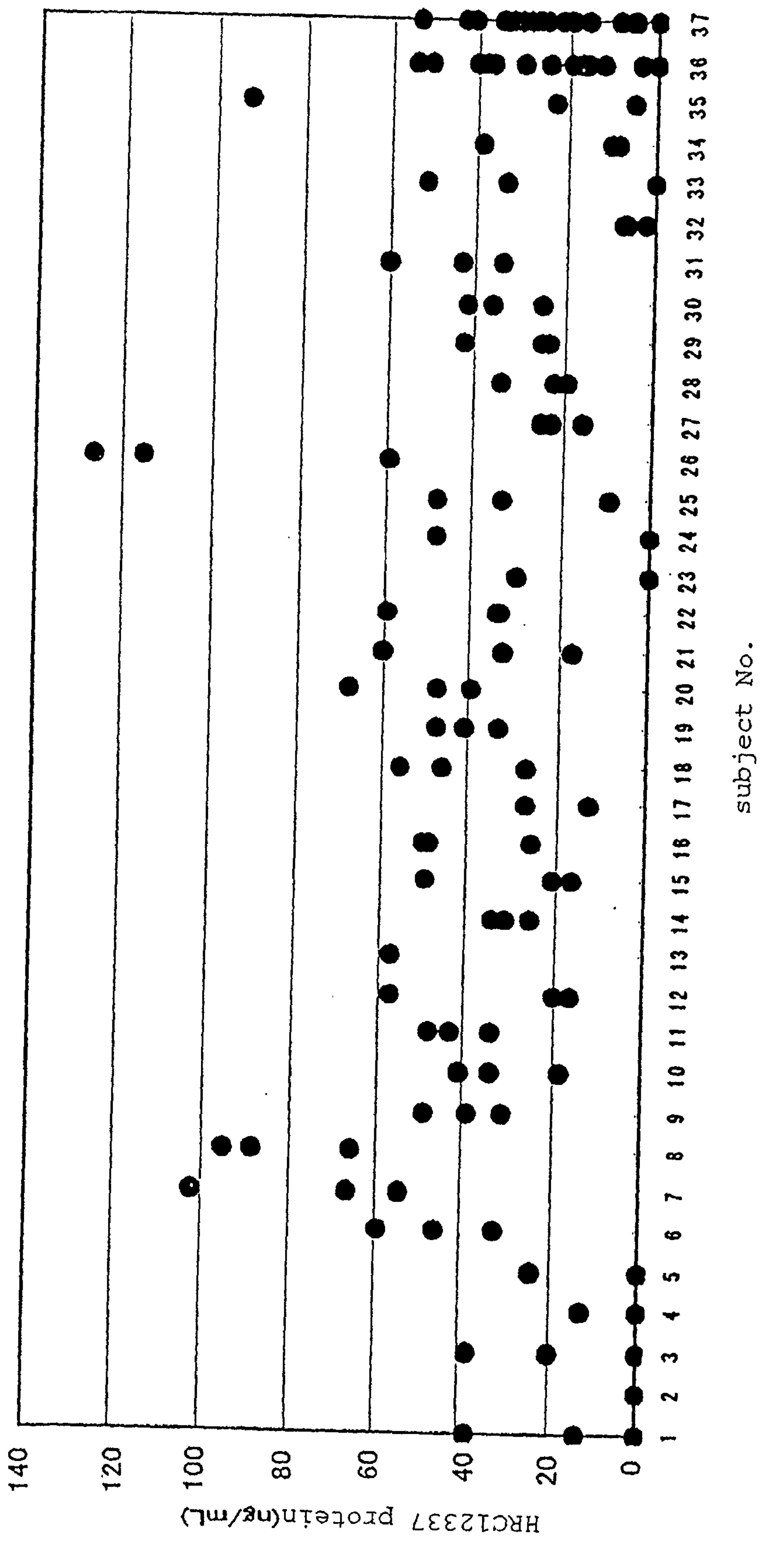
HRC12337 protein(ng/mL)
0
20
40
60
80
100
120
140
subject No.
1 2 3 4 5 6 7 8 9 10 11 12 13 14 15 16 17 18 19 20 21 22 23 24 25 26 27 28 29 30 31 32 33 34 35 36 37

| subject | disease name | subject | disease name |
|---|---|---|---|
| No. 1 | angina pectoris | No. 21 | myeloma |
| 2 | acute myocardial infarction | 22 | prostatic cancer |
| 3 | heart failure | 23 | brain infarction |
| 4 | hypertension | 24 | dementia |
| 5 | cardiac disease | 25 | rheumatoid arthritis |
| 6 | bronchial asthma | 26 | angiitis |
| 7 | pollinosis | 27 | autoimmune disease |
| 8 | atopic dermatitis | 28 | arrhythmia |
| 9 | pulmonary tumor | 29 | ovarian cancer |
| 10 | Sjogren's syndrome | 30 | acute leukemia |
| 11 | biliary calculus | 31 | chronic leukemia |
| 12 | hepatic failure | 32 | renal failure |
| 13 | autoimmune hepatitis | 33 | glomerular nephritis |
| 14 | hepatocirrhosis | 34 | prostatic hyperplasia |
| 15 | pancreatitis | 35 | Arzheimer's senile dementia |
| 16 | type II diabetes | 36 | healthy male |
| 17 | hyperlipemia | 37 | healthy female |
| 18 | osteoporosis | | |
| 19 | Grave's disease | | |
| 20 | Cushing's syndrome | | |

Fig. 8

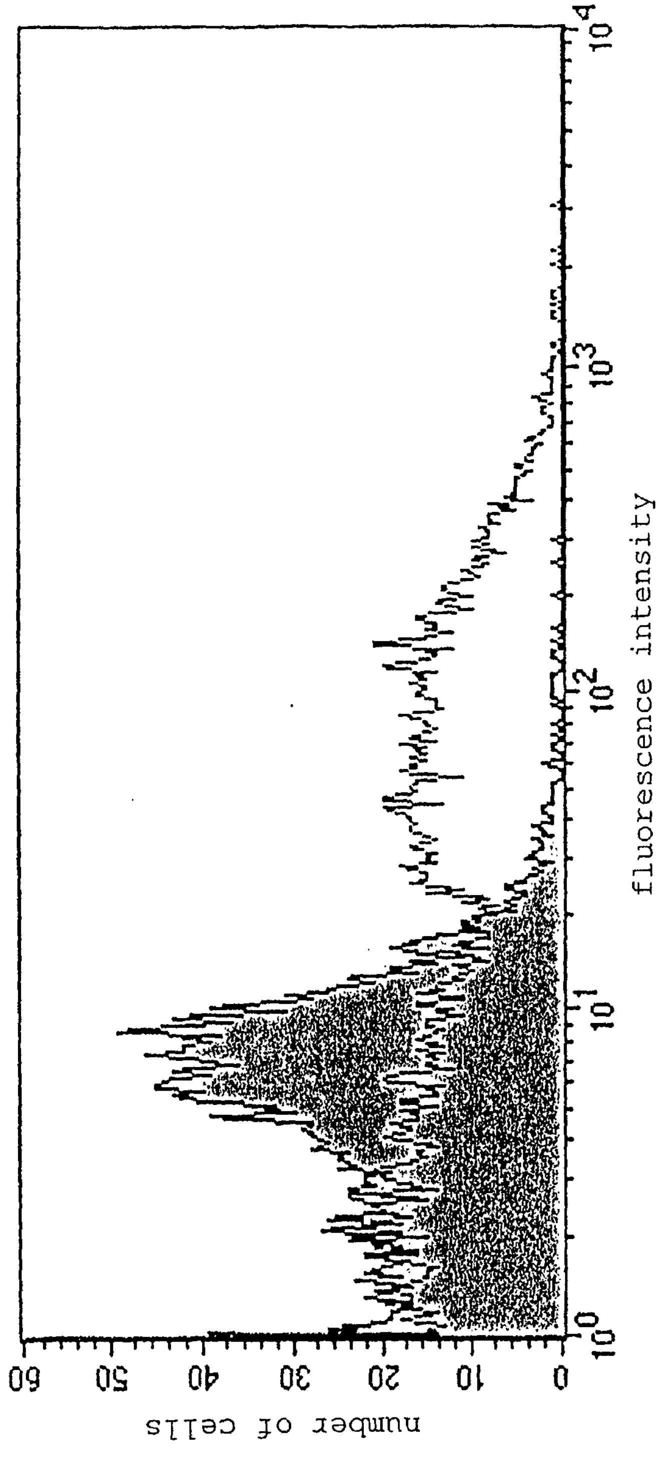
number of cells
0
10
20
30
40
50
60
fluorescence intensity
$10^0$
$10^1$
$10^2$
$10^3$
$10^4$

Fig. 9

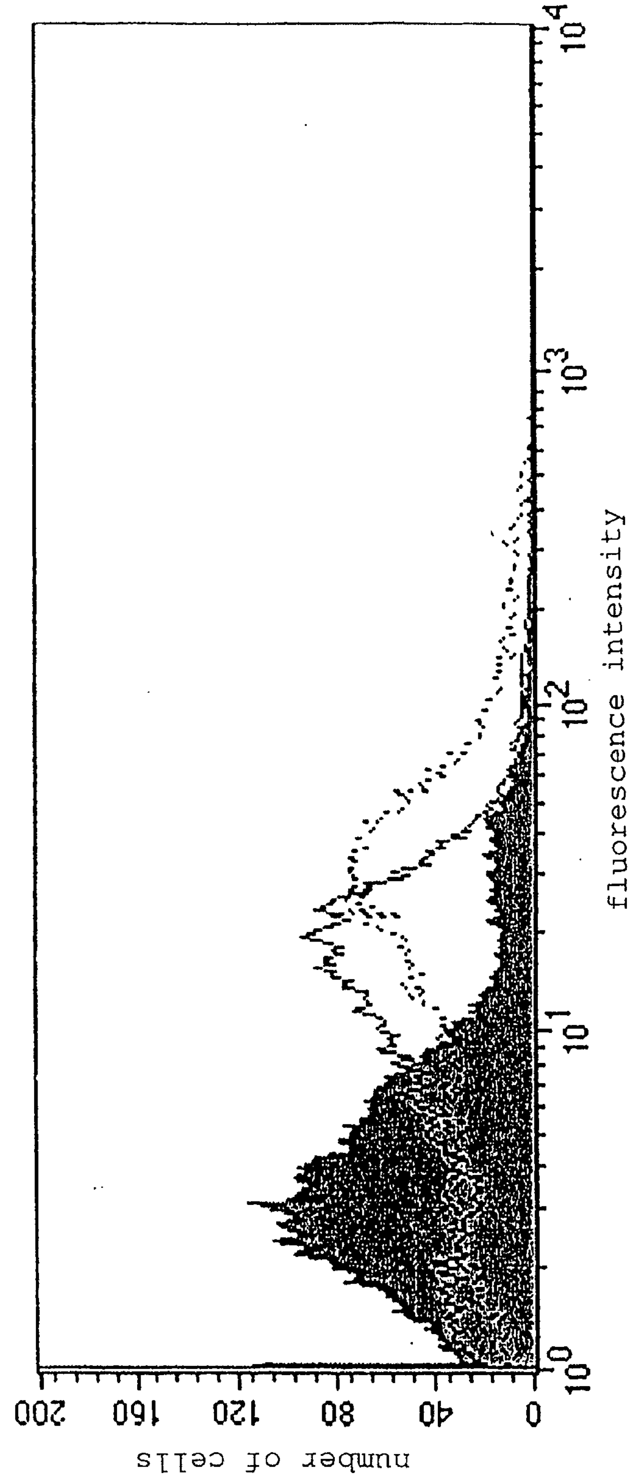
number of cells
0
40
80
120
160
200
fluorescence intensity
$10^0$
$10^1$
$10^2$
$10^3$
$10^4$

Fig. 10

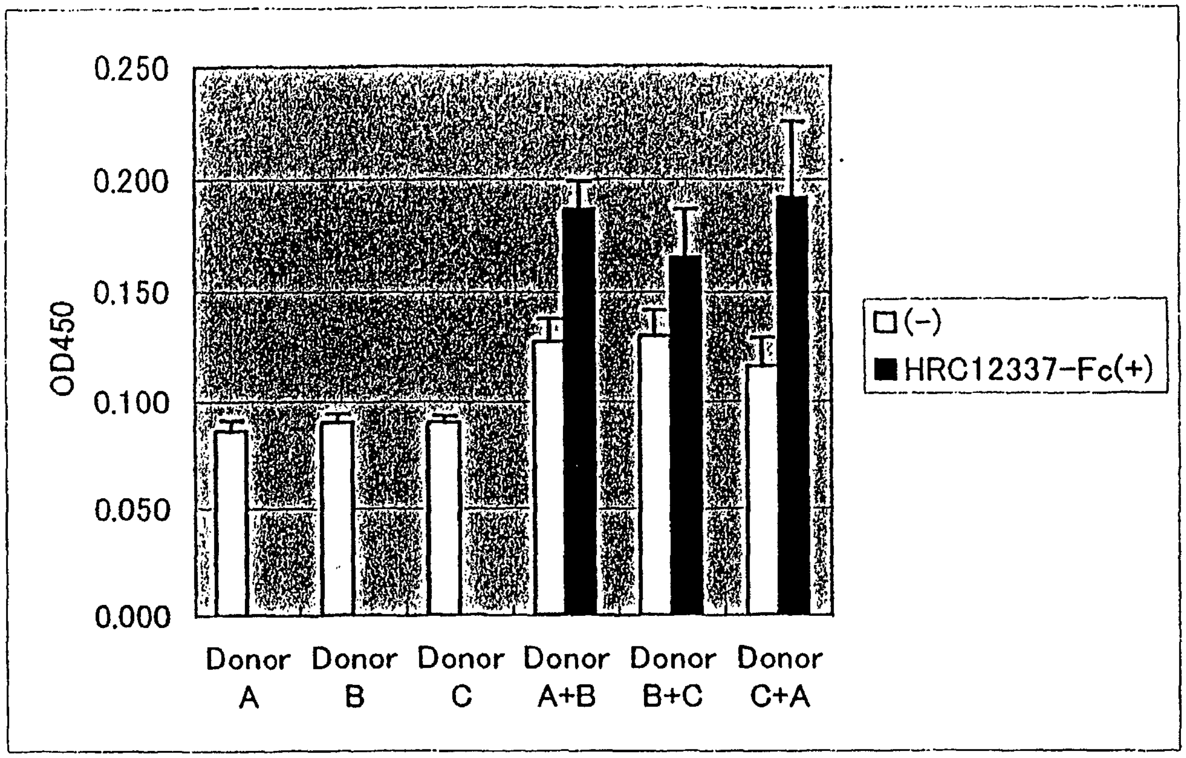
0.250
0.200
0.150
0.100
0.050
0.000
OD450
Donor A
Donor B
Donor C
Donor A+B
Donor B+C
Donor C+A
(-)
HRC12337-Fc(+)

Fig. 11

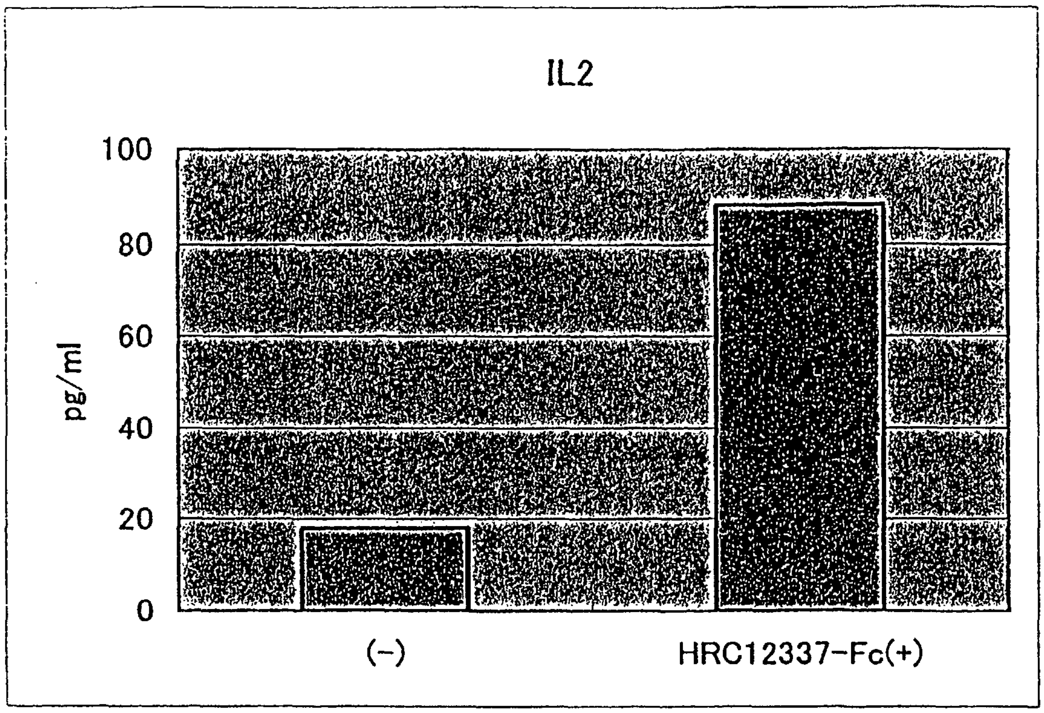

## INTERNATIONAL SEARCH REPORT

International application No.
PCT/JP02/01321

**A. CLASSIFICATION OF SUBJECT MATTER**

Int.Cl[7] C12N15/09, C12N15/12, C07K14/47, C07K16/18, C12N1/15, C12N1/19, C12N1/21, C12N5/10, C12Q1/02, A61K48/00, G01N33/50

**B. FIELDS SEARCHED**

Minimum documentation searched (classification system followed by classification symbols)

Int.Cl[7] C12N15/09, C12N15/12, C07K14/47, C07K16/18, C12N1/15, C12N1/19, C12N1/21, C12N5/10, C12Q1/02, A61K48/00, G01N33/50

Documentation searched other than minimum documentation to the extent that such documents are included in the fields searched

Electronic data base consulted during the international search (name of data base and, where practicable, search terms used)

SwissProt/PIR/GeneSeq, MEDLINE(STN),
GenBank/EMBL/DDBJ/GeneSeq, WPI(DIALOG), BIOSIS(DIALOG)

**C. DOCUMENTS CONSIDERED TO BE RELEVANT**

| Category* | Citation of document, with indication, where appropriate, of the relevant passages | Relevant to claim No. |
|---|---|---|
| A | Jiquan Q. ZHANG et al., Siglec-9, a Novel Sialic Acid Binding Member of the Immunoglobulin Superfamiry Expressed Broady on Human Blood Leukocytes. The Journal of Biological Chemistry, July 2000, Vol.275, No.29, pages 22121 to 22126 | 1-17 |
| A | Michela FALCO et al., Identification and Molecular Cloning of p75/AIRM1, A Novel Member of the Sialoadhesin Family That Functions as an Inhibitory Receptor in Human Natural Killer Cells. J.Exp.Med. September, 1999, Vol.190, No.6, pages 793 to 801 | 1-17 |
| A | Ann L. CORNISH et al., Characterization of Siglec-5, a Novel Glycoprotein Expressed on Myeloid Cells Related to CD33. Blood September, 1998, Vol.92, No.6, pages 2123 to 2132 | 1-17 |

☐ Further documents are listed in the continuation of Box C. ☐ See patent family annex.

* Special categories of cited documents:
"A" document defining the general state of the art which is not considered to be of particular relevance
"E" earlier document but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| | |
|---|---|
| Date of the actual completion of the international search<br>15 March, 2002 (15.03.02) | Date of mailing of the international search report<br>26 March, 2002 (26.03.02) |
| Name and mailing address of the ISA/<br>Japanese Patent Office<br><br>Facsimile No. | Authorized officer<br><br>Telephone No. |

Form PCT/ISA/210 (second sheet) (July 1998)